# EUROPEAN PATENT SPECIFICATION

(11) **EP 1 974 032 B1**
(45) Date of publication and mention of the grant of the patent: **21.12.2016**
(21) Application number: 06808713.9
(22) Date of filing: 30.11.2006
(51) Int. Cl.: C12N 15/06, G01N 33/50

(54) **METHOD FOR IDENTIFYING A MODULATOR OF A CELL SIGNALLING**
VERFAHREN ZUR IDENTIFIZIERUNG EINES MODULATORS EINER ZELLSIGNALGEBUNG
METHODE POUR IDENTIFIER UN MODULATEUR D'UN SIGNAL CELLULAIRE

(30) Priority: 30.11.2005 GB 0526664
(43) Date of publication of application: 01.10.2008
(60) Divisional application: 11170593.5
(73) Proprietor: Progenitor Labs Limited, London EC4A 3TW (GB)
(72) Inventor: CHOO, Yen, London SW11 4HL (GB)
(74) Representative: Schlich, George
(86) International application number: PCT/GB2006/004483
(87) International publication number: WO 2007/063316

(56) References cited:
- WO-A-2004/031369
- DING SHENG ET AL: "A role for chemistry in stem cell biology" NATURE BIOTECHNOLOGY, vol. 22, no. 7, July 2004 (2004-07), pages 833-840, XP002425681 ISSN: 1087-0156
- DING SHENG ET AL: "Synthetic small molecules that control stem cell fate" PROCEEDINGS OF THE NATIONAL ACADEMY OF SCIENCES OF USA, NATIONAL ACADEMY OF SCIENCE, WASHINGTON, DC, US, vol. 100, no. 13, 24 June 2003 (2003-06-24), pages 7632-7637, XP002383478 ISSN: 0027-8424
- WU XU ET AL: "A small molecule with osteogenesis-inducing activity in multipotent mesenchymal progenitor cells" JOURNAL OF THE AMERICAN CHEMICAL SOCIETY, AMERICAN CHEMICAL SOCIETY, WASHINGTON, DC, US, vol. 124, no. 49, 2002, pages 14520-14521, XP003005264 ISSN: 0002-7863
- WU X ET AL: "Small molecules that induce cardiomyogenesis in embryonic stem cells" JOURNAL OF THE AMERICAN CHEMICAL SOCIETY, AMERICAN CHEMICAL SOCIETY, WASHINGTON, DC, US, vol. 126, no. 6, 18 February 2004 (2004-02-18), pages 1590-1591, XP002324807 ISSN: 0002-7863
- CHOO Y: "Embryonic stem cell differentiation using combinatorial cell culture." CYTOTHERAPY, vol. 8, no. Suppl. 1, 2006, page 189, XP009080792 & 12TH ANNUAL MEETING OF THE INTERNATIONAL-SOCIETY-FOR-CELLULAR-THERAPY ; BERLIN, GERMANY; 2006, ISSN: 1465-3249
- DING ET AL: "A Combinatorial Scaffold Approach toward Kinase-Directed Heterocycle Libraries" JOURNAL OF THE AMERICAN CHEMICAL SOCIETY, AMERICAN CHEMICAL SOCIETY, WASHINGTON, DC, US, vol. 124, no. 8, 2002, pages 1594-1596, XP002210160 ISSN: 0002-7863
- DING SHENG ET AL: "Small molecules and future regenerative medicine" CURRENT TOPICS IN MEDICINAL CHEMISTRY, vol. 5, no. 4, May 2005 (2005-05), pages 383-396, XP009080879 ISSN: 1568-0266

## Description

### Field of the Invention

The disclosure relates to a method for identifying a modulator of a cell signaling pathway, in particular a method for identifying a modulator of a cell signalling pathway that triggers differentiation in cells. Modulators may be identified by exposing progenitor cells to candidate modulators. In one aspect, the progenitor cells are multipotent or unipotent stem cells which are isolated at various stages of differentiation, and further cultured together with a potential modulator of the differentiation process that acts by promoting or inhibiting the effect of cell signalling pathways on differentiation.

### Background to the Invention

The field of regenerative medicine holds the realistic promise of regenerating damaged tissues and organs *in vivo,* in patients with conditions such as cardiovascular disease, neurodegenerative disease, musculoskeletal disease, liver disease or diabetes. Techniques for regeneration of damaged tissue involve either the repair of existing diseased tissue in vivo using regenerative drugs or by replacement of such tissue using cells first prepared in vitro with or without the use of regenerative drugs and then transplanted in vivo.

In either case, the goals of regenerative medicine can only be realised if specific genes, factors or modulators controlling cell signalling pathways and the downstream cellular processes they regulate can be identified. It is thought that the controlled differentiation of stem cells in vitro for example, may provide a source of replacement cells for transplantation. The pluripotency and- plasticity of stem cells allows them to be committed to a particular cell type following- treatment with certain culture conditions. However such an approach relies on the prior identification of factors or modulators that control the cellular and molecular events of lineage differentiation. Use of these factors or modulators on stem cells ex vivo could reduce the likelihood of spontaneous differentiation of stem cells into divergent lineages upon transplantation, as well as reduce the risk of teratoma formation in the case of embryonic stem cells.

Such factors or modulators could also form the basis of therapies that aim to mobilise endogenous stem cells in vivo, or to trigger their differentiation into a cell type that can amplify, repair, restore, replace or otherwise benefit a damaged tissue. An example df a factor that affects cell differentiation and is used in therapy is erythropoietin (EPO). EPO is a naturally occurring protein factor that promotes the differentiation of haematopoietic precursors into erythrocytes. Recombinant EPO is used to treat anaemia and has a global market of approximately US$10 billion.

In addition to naturally occurring molecules or factors, it is possible to affect differentiation of cells using synthetic modulators of signalling pathways, in particular modulators of pathways controlling differentiation. SB-497115 is a small molecule drug being developed by GSK that mimics the activity of thrombopoietin (TPO), a protein factor that promotes growth and production of blood platelets. The drug could be used to treat thrombocytopenia: the inability to produce platelets, which are critically required as components of the clotting process during bleeding. It is estimated that the market opportunity is approximately US$4-5 billion.

Though regenerative drugs such as EPO or SB-497115 act on stem cells, a general method for the discovery of regenerative drugs using stem cells, in particular embryonic or foetal stem cells, has not been proposed.

Naive attempts at using stem cells in drug discovery have involved experiments in which pluripotent embryonic stem cells, self-renewing adult stem cells or cell lines have been used in cell-based phenotypic and pathway-specific screens of natural products or synthetic compounds to discover agents capable of effecting differentiation of these cells (see review by Ding & Shultz (2004) Nature Biotechnology 22: 833-840). One of the reasons for using these cells is that they can be easily amplified to yield the quantities required for a screen. However the approaches in the prior art, in which self-renewing, undifferentiated stem cells or cell lines are used, are found lacking as drug discovery methodologies for a number of reasons discussed below.

Firstly, the cell types used in the prior art (particularly ES cells and cell lines) may not be physiologically relevant targets suitable for pharmacological intervention in vivo. For example, while a factor which is able to cause differentiation of ES cells to e.g. cardiomyocytes (Wu et al., J Am Chem Soc. 2004:126(6):1590-1) may be of use in allowing the creation of cardiomyocytes in vitro from ES cells, the ES cell is not present in the adult and any agent which has activity specifically on the ES cell would likely not act as a regenerative medicine in vivo.

Secondly the cell types used in the prior art (particularly ES cells and self-renewing adult stem cells) lie too far upstream of the target cell lineage in the developmental pathway to undergo directed differentiation to that lineage in response to a single application of a single agent. For instance it is known that timed application of numerous factor cocktails in series are required to differentiate ES cells into specific lineages, particularly those which are specified relatively late in development as a result of a relatively complicated process of tissue specification.

Thirdly, the cell types used in the prior art (particularly primary adult stem cells) may be difficult to obtain in sufficient quantities to carry out large scale high-throughput drug screening.

Finally, the cell types used in the prior art (particularly primary adult stem cells) may exhibit highly variable effects in response to drugs, depending on the source or the method of isolation and preparation.

WO2004/03136 describes methods and cell signalling pathways which permit differentiation of cells - such as stem cells. In the technique described in WO2004/031369 cells are cultured under multiple culture steps under a plurality of conditions to modulate cellular pathways and the method provides a means of determining the effect of diverse multiple culture step regimes on cellular processes such as differentiation.

Existing approaches to discovering regenerative drugs are suboptimal and there exists a need for improved methods to discover such drugs. The present invention offers solutions to these problems and provides improved methods for the discovery of drugs -such as regenerative drugs.

### Summary of the Invention

The present invention is based, at least in part, on the finding that it is possible to arrest stem cells in a differentiation pathway thus isolating cells of another type suitable for screening. In one embodiment, this is achieved by obtaining cells of a first type and determining a differentiation protocol which leads to the appearance of a given target phenotype of interest via a progenitor cell, and optionally further modifying this protocol, typically by varying the cell culturing media, such that the differentiation process is stalled at a stage in which cells of another type, preferably progenitor cells, are present. The identity of the cells of the second type eg. progenitor cells need not be known but their existence in the preparation can be inferred from the fact that the phenotype of interest will appear if the original differentiation protocol is followed to completion. Thus, in one embodiment, the methods described herein can be used to produce developmental progenitors of cell types whose progenitors *in vivo* are not yet known.

One way of prpducing such cells is to use the method of WO2004/031369 in order to discover a series of culture steps leading to differentiation and subsequently modifying or truhcating the process leading to the isolation of progenitor cells. The invention recognises that by sequential exposure to selected agents cells may be isolated in a partially differentiated state which is substantially identical to the progenitor pool *in vivo,* and then screened for factors that induce further differentiation.

The present disclosure also recognises that regenerative drug discovery screening assays are more likely to identify effective drug candidates if a physiologically relevant progenitor cell is used in a cell based screen of natural products or synthetic compounds. The physiologically relevant progenitor cells have less developmental potential compared to self-renewing embryonic or adult stem cells and lie downstream of these multipotent cells in the developmental pathway. For example, the target cell type for EPO in the clinical treatment of anaemia is not the embryonic stem cell, nor the self-renewing adult haematopoietic stem cell, but rather a more committed erythrocyte progenitor cell which lies further along the developmental pathway and whose developmental potential is far more restricted (Fisher, Exp. Biol Med 2003 Jan;228(1):1-14). If this progenitor population were available for use in a drug discovery screening assay, the effect of EPO on regenerating erythrocytes would be readily apparent. On the other hand, this regenerative effect of EPO is not as readily apparent if undifferentiated ES cells are used in a screen, even though these cells can be differentiated to erythrocytes using suitable protocols. Therefore a physiologically relevant progenitor cell, rather than a multipotent self-renewing stem cell, should be used in a drug screen to identify regenerative drugs.

However an important problem of using progenitor populations is that they are extremely difficult to source, they have limited amplification capacity and in some instances they are completely uncharacterised. For instance, certain neural progenitors are well know but reside in the living brain and are thus difficult to source for drug screening assays. Furthermore, the liver is an organ capable of rapid regeneration in vitro, and liver biopsies are relatively easier to source compared to brain biopsies, but the progenitor cell population responsible for liver regeneration has nut yet been conclusively identified and therefore cannot be isolated, cultured and used in drug screening assays.

The present disclosure also recognises that physiologically relevant progenitor cells suitable for regenerative drug discovery screening assays can be derived from self-renewing embryonic dr adult stem cells, if these cells can be made to differentiate up to, but no further than, the relevant progenitor stage. There is therefore a need in the art for improved techniques to isolate partially differentiated cells in order subsequently to screen for factors that could be used to modulate their differentiation.

The present disclosure involves methods of isolating partially differentiated cell types which comprise physiologically relevant progenitor cells. In one embodiment, this is achieved by obtaining stem cells and subjecting them to the techniques described in in WO2004/031369 to dispover a differentiation protocol, and further modifying this protocol such that the differentiation process is stalled at, or not progressed past, a stage in which target progenitor cells are present. In another embodiment, it involves isolating them from the adult stem cell pool, developing foetus or animal in various stages of development and optionally modifying them such that they can be amplified *in vitro.* In addition, these progenitor cells may be used in assays in which natural products and candidate small molecule modulators of cell signaling are screened to identify agents which affect cell signalling in the progenitor cells, causing, for example, mobilization, amplification or differentiation, and which can then be developed into regenerative medicines

### Aspects and embodiments of the present invention

In a first aspect, there is provided a method for identifying a potential modulator of a cell signalling pathway, comprising the steps of: (a) providing a cell of a first cell type, wherein said first cell type may be differentiated to a second cell type via a progenitor cell by sequentially exposing said first cell type to one or more, preferably two or more reaction conditions; (b) adding to or replacing at least one of said two or more reaction conditions to which the progenitor cell has been exposed with exposure to one or more different reaction conditions comprising said potential modulator; and (c) monitoring the differentiation of the first cell type to determine formation of the second cell type.

In a further aspect, there is provided a method for identifying a potential modulator of a cell signalling pathways, comprising the steps of; (a) providing a cell of a first cell type, wherein said first cell type may be differentiated to a second cell type via a progenitor cell by sequentially exposing said first cell type to two or more reaction conditions; (b) adding to or replacing at least one of said two or more reaction conditions to which the progenitor cell has been exposed with exposure to one or more different reaction conditions comprising said potential modulator; and (c) monitoring the differentiation of the first cell type to determine formation of the second cell type, wherein differentiation of the cells to the second cell type is indicative that said potential modulator modulates the cell differentiation pathway.

The disclosure also recognises that by derivation from an organism in an early state of development, cells may be isolated in a partially differentiated state and optionally amplified, optionally further differentiated, and then screened for modulators that induce differentiation to a target cell lineage or phenotype

Accordingly in one embodiment, the first cell type is obtained or obtainable from an embryo or foetus and optionally modified to allow amplification.

In one embodiment, the progenitor cell is derived *in vitro* from a first cell type by exposure (eg. sequential exposure) to one or more (eg. two or more) reaction conditions.

In another embodiment, the cells are monitored for cell death instead of cell differentiation. Accordingly, the screen may be a toxicity screen.

In one embodiment, the reaction conditions comprise a screen of potential modulators (e.g. a screen of at least 100 potential modulators, at least 1000 potential modulators, or at least 10,000 potential modulators).

In another embodiment, the first cell type is a self-renewing stem cell.

The invention employs cell units. Such units may be single cells, but are advantageously colonies of two or more cells, which are arranged in such a form that they are resistant to disruption even during split pool culturing procedures. For instance, the cells may be cultured on a solid substrate, such as beads, as described in more detail below. In the present invention cell units can be isolated at any stage of the differentiation process triggered by the sequential addition of agents into the culture medium. Accordingly, there is provided a method for determining the effect of a plurality of culture conditions on a cell as described above, wherein cell units that are partially differentiated are then isolated and used in the method described for identifying the effect of modulators on cell signalling pathways.

Typically the cell units used are microcarriers which are small enough to be transferred to a HTS screening system in liquid phase and without substantial disruption of the cell unit. This system greatly facilitates the production of quantities of differentiated cells for screening, and also the set up of the HTS assay. In particular it also allows the use of cells for HTS without any prior disruption of the cells, such as by proteolytic digestion to allow transfer of cells from one vessel to another, which is advantageous since such processes may affect tHe immediate differentiation status or downstream differentiation ability of cells. Furthermore it allows the automated transfer of cells using robotic systems.

Thus an important advantage of using cell units - for example cell units grown on microcarriers to prepare the cellular material prior to screening is that it preserves the cellular niche that has arisen in the preparation process and which may be important for the downstream differentiation screen. If the cellular material were prepared in the conventional way and disrupted for dispensing into wells, then the niche would be disrupted. If on the other hand the cellular material were prepared for the screen in separate wells, then the resulting well-to-well variation in the preparation would make drug screening difficult to interpret.

Advantageously, in certain applications the cell units may be labelled. Labelling allows the following of the culture conditions to which the cells have been exposed; thus, any given cell unit can have its label read in order to determine how it has been derived from the starter cell pool or culture. Labelling may take any of a variety of forms, including nucleic acid labels, radiofrequency encoded tags, microsphere tags, barcoded tags and spatial encoding of cell units on a surface or matrix.

The label may be selected from the group consisting of a virus, an oligonucleotide, a peptide, a fluorescent compound, a secondary amine, a halocarbon, a mixture of stable isotopes, a bar code, an optical tag, a bread, a quantum dot and a radiofrequency encoding tag. Two or more labels may even be selected from this group and used in combination to label a cell unit, for instance a bead comprising fluorescent compounds and/or quantum dots. Labelling and specific labels to be used with cell units are further discussed in our co-pending application GB01517382.8.

Cells may be cultured in cell units, each cell unit comprising one or more cells. In another embodiment, the cell units are single cells. The cell unit may comprise one or more cells adherent to or bounded by a solid substrate. In a further embodiment, the solid substrate is a microcarriers or bead. In a still further embodiment the solid substrate is a well or medium-permeable barrier. The culture conditions may be media to which the cell is exposed. The media may contain one or more specific agents, Which influence a cellular process.

In one embodiment, the reaction conditions include any physical or chemical medium in which cells are isolated and manipulated but suitably the reaction condition is a culture condition to which cells are exposed. Culture conditions include growth media, temperature regimes, substrates, atmospheric conditions, physical cell handling and the like. Growth media comprise natural and synthetic substances that nourish and affect the cells including but not limited to basal media, growth factors, nutrients, buffers, chemicals, drugs and the like. The reaction conditions may even comprise a screen of potential modulators of a cell signalling pathway.

The invention may be used to monitor differentiation of a first cell type at any stage of development but the inventors recognise that due to their relative ease of culture, pluripotency and therapeutic potential, stem cells may be particularly suitable as a first cell type. Thus, in one- embodiment, the disclosure provides a methods as described above wherein the first cell type is a cell which has been arrested along a differentiation pathway between a stem cell and a differentiated cell type.

In one embodiment, the cell type is a primary cell, cell line or tumour derived cell line. The tissue of origin of the cell type may be selected from a group consisting of brain, heart, liver, lung, hair, eye, gut, blood, ear, kidney, skin, tooth, pancreas, muscle, bone and vasculature.

In another embodiment, there is provided for use of a partially differentiated, or progenitor, cell type. The said cell type may be isolated from an organism or produced from pluripotent cells using a method of determining the effect pf culture, conditions on differentiation; and further subjected to a method of identifying the effect of modulators on cell signalling pathways affecting differentiation. The method of identifying the effect of modulators on cell signalling pathways affecting differentiation may involve screening potential modulators using the drug discovery techniques commonly employed by pharmaceutical companies (Reinventing Drug Discovery, Executive Briefing, Accenture, 1997; High Performance Drug Discovery, Executive Briefing, Accenture, 2001).

In another embodiment, there is provided a method for exposing progenitor cells or partially differentiated cells to a potential modulator and then monitoring the effect of the modulator on the process of differentiation. In one embodiment, the potential modulator is an inhibitor of a cell signalling pathway, In another embodiment, the potential modulator is a promoter of a cell signalling pathway. The effect of a modulator to promote or inhibit cell signalling pathways affecting differentiation, may be assessed by a suitable assay including but not limited to monitoring phenotype, reporter gene expression, genotype, molecule production, viability, metabolic changes or the proliferative ability of cells.

The disclosure provides a method for obtaining progenitor cells or partly differentiated cells from tissues in developing embryos and foetuses, or indeed adults. As tissues develop through the foetal and adult stages, they develop stem cells which are progressively restricted in developmental capacity, ultimately becoming adult stem cells. Thus at any point in development of an organism, progenitor cells may be excised from tissues, for instance from the foetus of an animal or a human foetus obtained immediately following an elective abortion. For instance, cells which comprise the precursors to dopamine-producing cells may be isolated from specific regions of the developing central nervous system of the foetus. Since cells derived from foetal material are scarce, it may be necessary to amplify these cells. In this case it is possible to do this without affecting their differentiation state by transforming these cells, for instance using an oncogene such as c-myc. Suitably, the transformation is reversible and does not lead to differentiation of the progenitor cell. The disclosure recognises that foetal or adult stem cell material may require further differentiation in order to produce progenitor cells, and this can be achieved by the method disclosed below for other stem cells, such as ES cells.

The disclosure also provides a method for obtaining progenitor cells or partly differentiated cells from pluripotent cell lines, including but not limited to lines of embryonic stem cells, by determining a differentiation protocol and performing this in part. In this case the resulting cell population will comprise one or more progenitor cells: it is not necessary to know which proportion of the cells in the population are progenitor cells, nor to be able to identify these, however their presence may be inferred from the fact that fully differentiated cells would arise from these if the said differentiation protocol is concluded (instead of being arrested).
Differentiation protocols typically involve subjecting the cells to a temporally specified series of appropriate culture conditions. Cells giving rise to progenitor cells may be induced to differentiate along a desired developmental pathway using this method of serial cell culture. Cells may be arrested at any stage of that differentiation process, thus obtaining progenitor cells, by interrupting or modifying the series of appropriate culture conditions. For instance, if a ten day differentiation protocol comprising a series of five cell culture steps is required to differentiate ES cells to macrophages, then fully performing only three of the steps in this series will result in the partial differentiation of the ES cells along that lineage and will allow isolation bf macrophage progenitor cells.
The method described for identifying a plurality of culture conditions allows thousands or millions of cell culture conditions and reagents to be tested, in a multiplexed high-throughput assay, to determine the conditions necessary to achieve the differentiation of cells.

In another aspect there is provided a method for identifying modulators of cell signalling as previously described, wherein a first cell is differentiated to a second cell type by modulating cell signalling and/or the expression of one or more genes in the cell.
Cell signalling and/or the expression of the genes in the cell can be modulated by, for example, addition of biomolecules such as factors, growth factors, morphogens, hormones, receptor agonists and antagonists, lipids, antibodies, drugs and the like; or by addition of synthetic drugs, chemicals, small molecules and the like. Suitably, the above modulators are added in combinations, such as from a cell extract, from a co-culture, in animal serum, or a cocktail prepared *in vitro*.

Cell signalling arid/or the expression of a gene can also be modulated by transfecting or otherwise transferring a gene into the cell such that it is expressed or over expressed in a transient, ligand-induced or permanent manner. Alternatively, the expression of the endogenous gene may be altered, such as by targeted enhancer insertion or the administration of exogenous agents which cause an increase or decrease in expression of the gene. Moreover, the product of the gene may itself be administered to the cell, or its activity eliminated from the cell to achieve the same result. Modulators capable of decreasing the expression of a gene include interfering RNA or antisense compounds, while modulators capable of decreasing the activity of a protein include drugs, antibodies, aptamers and the like.
In one aspect the differentiation of the cell is monitored by observing the phenotype of the cell or detecting the modulation of expression of one or more genes in a cell, thereby determining the state of differentiation of said cell. Phenotype determination can be carried but by a variety of techniques, for instance by visual inspection of the cell units under a microscope, or using high content screening and analysis instrumentation (see Cellomics Inc; www.cellomics.com). Alternatively differentiation can be detected by observing a marker product characteristic of the differentiated cell. This may be an endogenous marker such as a particular DNA or RNA sequence, or a cell protein which can be detected by a ligand, conversion of an enzyme substrate, or antibody that recognises a particular phenotypic marker. A differentiation marker may also be exogenous, i.e. one that has been introduced into the cell population, for example by transfection or viral transduction. Examples of exogenous markers are the fluorescent proteins (e.g. GFP) or cell surface antigens which are not normally expressed in a particular cell lineage or which are epitope-modified, or from a different species. A transgene or exogenous marker gene with associated transcriptional control elements can be expressed in a manner that reflects a pattern representative of an endogenous gene(s) indicative of phenotype or differentiation state. This can be achieved by associating the gene with a cell type-specific promoter, or by integrating the transgene into a particular locus (e.g. see European patent No. EP 0695351). The markers indicative of differentiation may be detected by a variety of techniques, both manual and automated, including observation under a microscope, affinity purification ('planning'), or by fluorescence activated cell sorting (FACS). Accordingly, the present disclosure provides a method of monitoring differentiation wherein the modulation of expression of one or more reporter genes is observed wherein the reporter gene(s) respond(s) to one or more differentiation states of said cell.
In a further embodiment, the expression of genes involved is monitored on a gene chip. Gene Expression may conveniently be analysed using a gene chip or array technology, which is widely available from suppliers such as Affymetrix.
Advantageously, the genes employed in this analysis encode extracellular markers, which may be detected for instance by immunoassay.
In another embodiment of the invention the differentiation of a cell is monitored by loss of proliferative ability.

The disclosure moreover provides methods of culturing stem cells, and differentiated cells derived from stem cells in vitro, adherent to microcarriers, such as beads. Microcarrier culture has significant advantages, including the scale-up of cultures, and also allows units of stem cells to be exposed to selected culture conditions as required in order to obtain the desired growth and/or differentiation conditions.
The potential modulator may comprise an organic or inorganic small molecule, a natural or derivatised carbohydrate, protein, polypeptide, peptide, glycoprotein, nucleic acid, DNA, RNA, oligonucleotide or protein-nucleic acid (PNA). In another, embodiment, the potential modulator is obtained from a library of small molecules with drug like properties.
In a further aspect, there is- provided a modulator of a cell signalling pathway obtained or obtainable by the methods described herein.
In another aspect there is provided a pharmaceutical composition comprising the modulator together with a pharmaceutically acceptable carrier; diluent or expient.
In a further aspect, there is provided a partially differentiated cell, which has been differentiated *in vitro* from a stem cell and arrested along a differentiation pathway between a stem cell and a differentiated cell type. The partially differentiated cell may be a neuronal or haematopoietic cell. The partially differentiated cell may be a bipotent cell. The partially differentiated cell may be a unipotent cell.

In a further aspect, there is provided a method for identifying a modulator of a cell signalling pathway (eg. a regenerative drug) comprising the use of a progenitor dell.

In a further aspect, there is provided a method for identifying a modulator of a cell signalling pathway (eg. a regenerative drug) comprising the use of a partially differentiated cell, which has been differentiated *in vitro* from a stem cell and arrested along a differentiation pathway between a stem cell and a differentiated cell type

In a further aspect, there is provided the use of a progenitor cell in a drug screening assay to identify a modulator of a cell signalling pathway (eg. a regenerative drug).

In a further aspect, there is provided the use of a partially differentiated cell, which has been differentiated *in vitro* from a stem cell and arrested along a differentiation pathway between a stem cell and a differentiated cell type in a drug screening assay to identify a rhodulator of a cell signalling pathway (eg. a regenerative drug).

In a further aspect, there is provided a method for differentiating an embryonic stem cell into a progenitor of the myeloid lineage, comprising the use of a gelatin microcarrier (eg. a CultiSpher microcarrier).

In a further aspect, there is provided the use of a gelatin microcarrier (eg. a CultiSpher microcarrier) for differentiating embryonic stem cells into haematopoietic progenitors.

In a further aspect, there is provided a method for producing a haematopoietic cell from a stem cell *in vitro* comprising exposing said stem cell to one or more, preferably, two or more, reaction conditions, wherein said reaction conditions comprise incubating said stem cell with: (a) retinoic acid, dimethylsulphoxide (DMSO) and/or stem cell factor (SCF); and (b) insulin, stem cell factors (SCF), TGF beta 1, BMP2, BMP4 and/or TPO; and (c) IL-3, IL-6, TPO, EPO and/or M-CSF.

In one embodiment, the stem cell is seeded on a microcarrier- such as a gelatin microcarrier.

In one embodiment, the stem cell is contained in an IMDM basal medium or a Streamline Haematopoietic Expansion Medium.

In one embodiment, in step (b) insulin alone is used.

In one embodiment, in step (b) SCF, TGF beta 1, BMP2 and TPO is used.

In one embodiment, in step (c) IL-3 and IL-6 are used. In another embodiment, TPO, EPO and/or M-CSF are also used.

In one embodiment, the step (a) is performed on day 1.

In one embodiment, the step (b) is performed on day 4.

In one embodiment, the step (c) is performed on day 6.

### Detailed Description of the Invention

### Definition

**Culture Conditions** As used herein, the term "culture conditions" refers to the environment which cells are placed in or are exposed to in order to promote growth or differentiation of said cells. Thus, the term refers to the medium, temperature, atmospheric conditions, substrate, stirring conditions and the like which may affect the growth and/ or differentiation of cells. More particularly, the term refers to specific agents which may be incorporated into culture media and which may influence the growth and/or differentiation of cells.

**Cell** A cell, as referred- to herein, is defined as the smallest structural unit of an organism that is capable of independent functioning, or a single-celled organism, consisting of one or more nuclei, cytoplasm, and various organelles, all surrounded by a semipermeable cell membrane or cell wall. The cell may be prokaryotic, eukaryotic or archaebacterial. For example, the cell may be a eukaryotic cell. Mammalian cells are suitable, especially human cells, Cells may be natural or modified, such as by genetic manipulation or passaging in culture, to achieved desired properties. A **stem cell** is defined in more detail below, and is a totipotent, pluripotent or multipotent cell capable of giving rise to more than one differentiated cell type. Stem cells may be differentiated *in vitro* to give rise to differentiated cells, which may themselves be multipotent, or may be terminally differentiated. Cells differentiated *in vitro* are cells which have been created artificially by exposing stem cells to one or more agents which promote cell differentiation.

**First cell type** in one embodiment, the first cell type is a cell that retains the ability to renew itself through cell division and can differentiate into a wide range of specialized cell types. In another embodiment, the first cell type is a cell that is less differentiated than a progenitor cell. In another embodiment, the first cell type is a stem cell, as described herein below. The stem cell may be, for example, an embryonic stem cell or an adult stem cell. The cells of the first type may be differentiated into a certain lineage of a second cell type before the cells of the second cell type are screened. Thus, in one embodiment, the first cell type is differentiated to a second cell type by exposing (eg. sequentially exposing) the first cell type to two or more (eg. three or more, four or more, or five or more) reaction conditions.

**Second cell type** In one embodiment, the second cell type is cell that can only differentiate, but it cannot renew itself anymore. The second cell type may be more limited in the kinds of cells it can become than the first cell type. The second cell may be more differentiated than the first cell type. The second cell may be more differentiated than the progenitor cell. In one embodiment, the second cell type is a partially differentiated cell, which has been differentiated *in vitro* from the first cell type and arrested along a differentiation pathway between a cell of the first type (eg. a stem cell) and a differentiated cell type. In another embodiment, the second cell type is a cell with a target cell phenotype.

**Regenerative medicine or drug** The term 'regenerative drug' refers to a natural or synthetic substance which-acts on a stem cell or progenitor cell and is thus able to regenerate or repair a tissue or organ of the body. 'Regenerative medicine' refers to the same, or to the discipline of regenerating tissues or organs as a medical treatment. Regenerative medicine encompasses cell replacement therapies, and/or the administration of regenerative drugs to patients.

**Progenitor** A progenitor or 'progenitor cell' is a cell type which lies upstream of a more differentiated cell, but downstream of a true stem cell. Progenitors are not typically capable of long term self-renewal as are true stem cells, and their developmental potential is more limited than is that of stem cells. For instance CFU-E and BFU-E are erythrocyte-committed progenitor cell populations, whereas the LT-HSC is a self-renewing and multipotent haematopoietic stem cell and the ES cell is a self-renewing arid pluripotent stem cell. Differentiated erythrocytes can be derived from CFU-E which in turn can be derived from LT-HSC which in turn can be derived from ES cells.

**Cell signalling** The term "cell signalling" refers to the molecular mechanisms whereby cells detect and respond to external stimuli and send messages to other cells. Cell signalling therefore includes transcriptional and translational controls and mechanisms as well as signal transduction mechanism.

**Modulator** The term "modulator" refers to any factor that can vary the state of a cell, changing it from one state to another. In the context of the invention this refers to modulation of cell signalling processes. Modulators may inhibit or promote particular cell signalling pathways. They may take the form of natural products or chemically synthesised molecules; for example, an organic or inorganic small molecule, a natural or derivatised carbohydrate, protein, polypeptide, peptide, glycoprotein, nucleic acid, DNA, RNA, oligonucleotide or protein-nucleic acid (PNA). Modulators also include agonists or antagonists. Modulators that are inhibitors include but are not limited to: nonspecific, irreversible, reversible - competitive and noncompetitive inhibitors. Modulators that promote cell signalling stimulate or enhance the effect of a particular molecular pathway on the cell and include but are not limited to: agonists, agonist mimetics, co-factors, promoters and the like.

**Amplification** "amplification" refers to a process by which an increase in magnitude or number of cells, cellular components or cellular processes occur. In particular amplification refers to a process of increasing cell numbers in a cell culture system. This may occur by increasing the rate of proliferation or survival of cells in the system.

**Compound** The term "compound" is used herein in accordance with the meaning normally assigned thereto in the art. The term compound is used in its broadest sense i.e. a substance comprising two or more elements in fixed proportions, including molecules and supramolecular complexes. This definition includes small molecules (typically <500 Daltons) which make up the majority of pharmaceuticals. However, the definition also includes larger molecules, including polymers, for example polypeptides, nucleic acids and carbohydrates and supramolecular complexes thereof.

**High-throughput** screening The term "high- throughput screening refers to the large-scale, trial-and-error evaluation of compounds in a parallel target-based or cell-based assay.

**Compound library** A "compound library" is a group of diverse compounds that can be used to identify new lead candidates in the drug discovery process. Compound libraries may be generated by any means known in the art, including combinatorial chemistry, compound evolution, or purchased from commercial sources such as Sigma Aldrich, Discovery Partners International, Maybridge and Tripos. A repertoire advantageously comprises at least 10², 10³, 10⁴, 10⁵, 10⁶, 10⁷, 10⁸, 10⁹, 10¹⁰, 10¹¹ or more different compounds, which may be related or unrelated in structure or function.

**Modulation** The term modulation is used to signify an increase and/or decrease in the parameter being modulated. Thus, modulation of gene expression includes both increasing gene expression and decreasing gene expression.

**Cellular process** A cellular process is any characteristic, function, process, event, cause or effect, intracellular or extracellular, which occurs or is observed or which can be attributed to a cell. Examples of cellular processes include, but are not limited to, viability, senescence, death, pluripotency, morphology, signalling, binding, recognition, molecule production or destruction (degradation), mutation, protein folding, transcription, translation, catalysis, synaptic transmission, vesicular transport, organelle function, cell cycle, metabolism, proliferation, division, differentiation, phenotype, genotype, gene expression, or the control of these processes.

**Cell unit** A group of cells, which may be a group of one. Pools of cell units may be sorted, subdivided and handled without substantially dissociating the cell units themselves, such that the cell unit behaves as a colony of cells and each cell in the cell unit is exposed to the same culture conditions. For example, a cell unit may comprise a bead to which is adhered a group of cells.

**Totipotent** A totipotent cell is a cell with the potential to differentiate into any type of somatic or germ cell found in the organism. Thus, any desired cell may be derived, by some means, from a totipotent cell.

**Pluripotent or Multipotent** A pluripotent or multipotent cell is a cell which may differentiate into more than one, but probably not all, cell types.

**Label** A label or **tag,** as used herein, is a means to identify a cell unit and/or determine a culture condition, or a sequence of culture conditions, to which the cell unit has been exposed. Thus, a label may be a group of labels, each added at a specific culturing step; or a label added at the beginning or the experiment which is modified according to, or tracked during, the culturing steps to which the cell unit is exposed; or simply a positional reference, which allows the culturing steps used to be deduced. A label or tag may also be a device that reports or records the location or the identity of a cell unit at any one time, or assigns a unique identifier to the cell unit. Examples of labels or tags are molecules of unique sequence, structure or mass; or fluorescent molecules or objects such as beads; or radiofrequency and other transponders; or objects with unique markings or colours or shapes.

**Exposure to culture conditions** A cell is exposed to culture conditions when it is placed in contact with a medium, or grown under conditions which affect one or more cellular process(es) such as the growth, differentiation, or metabolic state of the cell. Thus, if the culture conditions comprise culturing the cell in a medium, the cell is placed in the medium for a sufficient period of time for it to have an effect. Likewise, if the conditions are temperature conditions, the cells are cultured at the desired temperature.

**Pooling** The pooling of one or more groups of cell units involves the admixture of the groups to create a single group or pool which comprises cell units of more than one background that is, that have been exposed to more than one different sets of culture conditions. A pool may be subdivided further into groups either randomly or non-randomly; such groups are not themselves "pools" for the present purposes, but may themselves be pooled by combination, for example after exposure to different sets of culture conditions.

**Proliferation** Cell growth and cell proliferation are used interchangeably herein to denote multiplication of cell numbers without differentiation into different cell types or lineages. In other words, the therms denote increase of viable cell numbers. In one embodiment, proliferation is not accompanied by appreciable changes in phenotype or genotype.

**Differentiation** Cell differentiation is the development, from a cell type, to a different cell type. For example, a bipotent, pluripotent or totipotent cell may differentiate into a neural cell. Differentiation may be accompanied by proliferation, or may be independent thereof. The term 'differentiation' generally refers to the acquisition of a phenotype of a mature celt type from a less developmentally defined cell type, e.g. a neuron, or a lymphocyte, but does not preclude transdifferentiation, whereby one mature cell type may convert to another mature cell type e.g. a neuron to a lymphocyte. In this application 'differentiation' will be taken to mean 'de-differentiation' and vice versa. Commonly, 'de-differentiation' refers to the acquisition of a phenotype of a less mature cell type from a more developmentally defined cell type, e.g. a myocyte becoming a myogenic precursor. De-differenciation may be followed-by further differentiation to revert to the original cell type, or to a further cell type [Ding & Shultz (2004) Nature Biotechnology 22: 833-840, and references therein].

**Differentiation state** The differentiation state of a cell is the level to which a cell has differentiated along a particular pathway or lineage.

**State of a cellular process** The state of a cellular process refers to whether a cellular process is occurring or not and in complex cellular processes can denote a particular step or stage in that cellular process. For example, a cellular differentiation pathway in a cell may be inactive or may have been induced and may comprise a number of discrete steps or components such as signalling events characterised- by the presence of a characteristic set of enzymes or intermediates.

**Gene** A gene is a nucleic acid which encodes a gene product, be it a polypeptide or an RNA gene product. As used herein, a gene includes at least the coding sequence which encodes the gene product; it may, optionally, include one or more regulatory regions necessary for the transcription and/or translation of the coding sequence.

**Gene Product** A gene product is typically a protein encoded by a gene in the conventional manner. However, the term also encompasses non-polypeptide gene products, such as ribonucleic acids, which are encoded by the gene.

**Nucleic acid synthesis** Nucleic acids may be synthesised according to any available technique. In one embodiment, nucleic acid synthesis is automated. Moreover, nucleic acids may be produced by biological replication, such as by cloning and replication in bacterial or eukaryotic cells, according to procedures known in the art.

**Differential Expression** Genes which are expressed at different levels in response to cell culture conditions can be identified by gene expression analysis, such as on a gene array, by methods known in the art. Genes which are differentially expressed display a greater or lesser quantity of mRNA or gene product in the cell under the test conditions than under alternative conditions, relative to overall gene expression levels.

**Transfection** Genes may be transected into cells by any appropriate means. The term is used herein to signify conventional transfection, for example using calcium phosphate, but also to include other techniques for transferring nucleic acids into a cell, including transformation, viral transduction, electroporation and the like.

### Cell-based assays

Cell-based assays are an important part of modern biomedical sciences and comprise any assay that involves a step in which a cell is used. Cell-based assays can be used across nearly all stages of the pharmaceutical drug discovery and development process, and are valuable in providing information about how a compound is likely to interact in a biological system, not just about how it interacts with a potential drug target in isolation.

For example, cell-based assays can be used to- identify and validate potential drug targets. Cell-based assays have been developed that can be used to identify genes or cellular pathways involved in disease processes, to determine the functions of target genes, or to measure phenotypic changes that may be induced upon activation of certain genes or their products.

Cell-based assays can also be used in drug discovery for lead-compound discovery, selection, and optimization. Unlike the biochemical assays that are often used in traditional high-throughput-screening assays, cell-based assays can provide information relating to drug properties such as absorption, permeability, selectivity, specificity, and metabolism. As a result, lead compounds that are selected after cell-based screening are better characterized, more likely to provide, valuable leads and less likely to be eliminated in subsequent phases of the drug discovery process.

A major application of cell-based assays is in toxicity screening. A crucial part of drug discovery and development is the screening of drug candidates to eliminate compounds that will cause side effects. However, current methodologies are- largely inadequate, and in particular the use of animal models for toxicity screening is expansive and time-consuming. In addition, animal models can be unreliable, because results in these models do not always accurately predict how a compound will perform in humans. Thus human cell-based screening is suitable.

### Screening assays and HTS

High throughput screens (HTS) can be used in the present invention in order to screen for new drug targets. The emphasis of pharmaceutical research activities has shifted toward the purposeful discovery of novel chemical classes and novel molecular targets. This change in emphasis, and timely technological breakthroughs (e.g molecular biology, laboratory automation, combinatorial chemistry) gave birth to high throughput screening, or HTS, which is now widespread throughout the biopharmaceutical industry.

High throughput screening involves several steps: creating an assay that is predicative of a particular physiological response; automating the assay so that it can be reproducibly performed a large number of times; and, sequentially testing samples from a chemical library to identify chemical structures able to "hit" the assay, suggesting that such structures might be capable of provoking the intended physiological response. Hits from the high throughput screen are followed up in a variety of secondary assays to eliminate artifactual results, particularly toxic compounds. Thus, the assays used in high throughput screens are intended to detect the presence of chemical samples (e.g. compounds, substances, molecules) possessing specific biological or biochemical properties. These properties are chosen to identify compounds with the potential to elicit a specific biological response when applied in vivo. High throughput screens identify both agents that can be used as drugs themselves and in addition, drug candidates that will ultimately be used as drugs. A compound of a certain chemical class that is found to have some level of desired biological property in a high-throughput assay can then be the basis for synthesis of derivative compounds. Cell based assays utilise intact cells in culture. Examples of such assay include luciferase reporter gene assays and calcium flux assays.

A particularly powerful method of performing a cell-based assay suitable for the identification of differentiation modulators and regenerative drugs, as described in the -present invention, is to determine the differentiation status of progenitor cells by monitoring a genetic marker of differentiation. The marker may be endogenous, such as an expressed nucleotide sequence or protein that is present specifically in the differentiated cell type but not in its progenitor, nor in any other cell present in the cell population used for screening. The marker may also be an exogenous marker (i.e. a reporter gene) which is introduced into the cell population used for screening in such a way that it is expressed specifically in the differentiated cell type but not in its progenitor, nor in any other cell present in the cell population used for screening. Examples of exogenous markers are enzymes such as Lac Z, or non-enzymic markers sUch as the fluorescent proteins (e.g. GFP; YFP etc), or cell surface antigens which are not normally expressed in a particular cell lineage or which are epitope-modified or from a different species. A transgene or exogenous marker gene with associated transcriptional control elements can be expressed in a manner that reflects a pattern representative of an endogenous gene(s). This can be achieved by associating the gene with elements of a cell type-specific promoter, or by integrating the transgene into a particular locus which is expressed in a cell-type specific manner (e.g. see European patent No. EP 0695351).

In one embodiment, the undifferentiated cell type used as the starting material is modified to express two markers/reporters (e.g. both GFP and YFP) such that one marker indicates the presence of the progenitor cell type submitted to the HTS process, and the other marker indicates the presence of the differentiated cell type produced by the addition of a suitable modulator.
A number of suitable reporter gene systems and cellular screening assays, including dual reporter systems, are disclosed in reviews (and references therein) by Hill et al. [Current Opinion in Pharmacology (2001) 1:526-532] and by Blake [Current Opinion in Pharmacology (2001) 1:533-539].

### Progenitor cell

A progenitor cell is any somatic cell which has the capacity to generate fully differentiated, functional progeny by differentiation and proliferation. Progenitor cells include progenitors from any tissue or organ system, including, but not limited to, blood, nerve, muscle, skin, gut, bone, kidney, liver, pancreas, thymus, arid the like.

Progenitor cells may be distinguished from differentiated cells (*ie.* those cells which may or may not have the capacity to proliferate, *i.e.,* self-replicate, but which are unable to undergo further differentiation to a different cell type under normal physiological conditions). Progenitor cells may be further distinguished from abnormal cells - such as cancer cells, especially leukemia cells, which proliferate (self-replicate) but which generally do not further differentiate, despite appearing to be immature or undifferentiated.

Progenitor cells include all the cells in a lineage of differentiation and proliferation prior to the most differentiated or the fully mature cell.

According to one embodiment of the present inventions a distinction is drawn between a 'stem cell' and a 'progenitor' cell. Stem cells are typically pluripotent and multipotent and can give rise to a number of lineages. Progenitor cells, and in particular lineage-committed progenitor cells, are only capable of producing the cells of a single lineage. Hence the developmental potential of progenitors is typically more restricted than that of stem cells, A second important difference between stem cells and progenitor cells is that stem cells are capable of significant amplification - under the correct culturing conditions they can divide indefinitely - whereas progenitor cells have a limited proliferation capacity. These differences mean that, for instance, it is possible to reconstitute the haematopoietic system of an animal using-haematopoietic stem cells but not haematopoietic progenitor cells.

By way of example, production of mature, functional red blood cells results from proliferation and differentiation of "unipotential progenitors," *i.e*., those progenitors which have the capacity to make only one type of one type of blood cell. Various other hematopoietic progenitors (HPCs) have been characterized.

HPCs consist of many subclasses including pluripotent stem cells, lymphoid stem cells, CFU-GEMM colony forming unit granulocyte, erythroid, macrophage or megakaryocyte, BFU-E, CFU-E, CPU-Meg, CFU-GM colony forming unit granulocyte or macrophage, CFU-EoS colony forming uniteosinophil, progenitor B cells and progenitor T cells.

### Stem Cells

Stem cells are described in detail in Stem Cells: Scientific Progress and Future Research Directions. Department of Health and Human Services. June 2001. http://www.nih.gov/news/stemcell/scireport.htm.

Stem cells are cells that are capable of differentiating to form at least one and sometimes many specialised cell types. The repertoire of the different cells that can be formed from stem cells is thought to be exhaustive; that is to say it includes all the different cell types that make up the organism. Stem cells are present throughout the lifetime of an organism, from the early embryo where they are relatively abundant, to the adult where they are relatively rare. Stem cells present in many tissues of adult animals are important in normal tissue repair and homeostasis.

The existence of these cells has raised the possibility that they could provide a means of generating specialised functional cells *in vitro* that can be transplanted into humans and replace dead or non-functioning cells in diseased tissues. The list of diseases for which this may provide therapies includes Parkinson's disease, diabetes, spinal cord injury, stroke, chronic heart disease, end-stage kidney, disease, liver failure and cancer. In order for cell replacement therapy to become feasible a number of major breakthroughs in stem cell research are required, including improvements in the growth of stem cells, differentiation of stem cells and avoidance of immunological rejection of stem cells.

For this reason, alternative approaches to exploiting stem cells for therapy are being considered. As described herein, methods are disclosed for the discovery of compounds which may be developed into drugs (*eg.* regenerative drugs) that cause endogenous stem cells to regenerate tissues of the body,

### Types of stem cell

There is still considerable debate about what constitutes a stem cell, however for the purposes of this discussion a key characteristic is the ability to differentiate into a different cell type. An optional characteristic is the ability to self-renew, in certain cases indefinitely, allowing amplification of the cell numbers. Examples of stem cells are given below.

Different stem cells have differing potential to form various cell types: spermatogonial stem cells are unipotent as they naturally produce only spermatozoa, whereas haematopoietic stem cells are multipotent, and embryonic stem cells are thought to be able to give rise to all cell types and are said to be totipotent or pluripotent.

To date three types of mammalian pluripotent stem cell have been isolated. These cells can give rise to cell types that are normally derived from all three germ layers of the embryo (endoderm, mesoderm and ectoderm). The three types of stem cell are; embryonal carcinoma (EC) cells, derived from testicular tumours; embryonic stem (ES) cells, -derived from the pre-implantation embryo (normally the blastocyst); and embryonic germ (EG) cells derived from the post-implantation embryo (normally cells of the foetus destined to become part of the gonads). These cells are receiving particular attention in the effort to direct differentiation, precisely because they are pluripotent:

Stem cells are also present in the adult organism. An adult stem cell is an undifferentiated cell that pccurs in a (differentiated (specialised) tissue, renew itself, and can differentiate to yield more specialised cells. Recently it has been shown that adult stem cells are capable of plasticity, that is to say they can differentiate to yield cell types that are not characteristic of the tissue in which they reside, nor indeed of the germ layer from which that tissue originates For example, it has been shown that blood stem cells (derived from mesoderm) can differentiate into neurons (normally derived from ectoderm). Toma et al. (2001, Nature Cell Biol. 3, p778-784) have recently described the identification and isolation of a new type of stem cell that was derived from the dermis of the skin. These stem cells Were termed skin-derived precursor (SKP) cells. The SKP cells could be induced to differentiate by culturing on poly-lysine and varying the concentration of serum in the culture medium. In the absence of serum they differentiate into neurons and glial cells; with addition of 3% serum they differentiate into smooth-muscle cells; and increasing the serum to 10% causes the SKP cells to differentiate into adipocytes. Adult stem cells have so far been reported in tissues as diverse as the nervous system, the bone marrow and blood, the liver, skeletal muscle, the skin and digestive system.

In addition to the adult stem cells there are numerous types of progenitor or precursor cells, as described herein. These are cells that are partially restricted in their differentiative potential and occur in probably all of the tissues of the body - they are capable of differentiating but differ from stem cells in that their repertoire is not as broad, and by definition they are not capable of self-renewal.

Recent evidence even suggests that differentiated cell types are capable of changing phenotype. This phenomenon, termed transdifferentiation, is- the conversion of one differentiated cell type to another, with or without an intervening cell divsion. It was previously generally accepted that the terminal differentiated state is fixed, but it is now clear that differentiation can sometimes be reversed or altered. In vitro protocols are now available in which cell lines can be induced to transdifferentiate (see Shen, Slack & Tosh, 2000, Nature Cell Biol .vol 2, p. 879-887; Horb et al, 2003, Current Biol. Vol 13, p105-115). Finally, there have been reports of specialised cell types that can de-differentiate to yield stem-like cells with the potential to differentiate into further cell types.

### Stem cell growth and differentiation

An important property of stem cells is their ability to divide symmetrically in culture, giving rise to two daughter cells that are exact copies of the stem cell from Which they were derived. This allows stem cells to be expanded in culture in their undifferentiated state, producing enough material for screening purposes, biological studies or even cell therapy. The means by which stem cells are able to do this is understandably the subject of intensive research, yet-few of the factors that promote stem cell renewal are known. Typically, pluripotent stem cell lines are maintained on mitotically inactive feeder layers of fibroblasts, or medium conditioned by such cells. It is assumed that feeder cells remove/neutralise some unknown factor from the culture medium, and/or they provide a factor that suppresses the differentiation and promotes the self-renewal of stem cells. One such factor is leukaemia inhibitory factor (LIF), a member of the cytokine family related to IL-6, which is capable of promoting mouse ES cell self-renewal in the absence of feeder cells. Stem cells grown in the absence of feeder cells (and/or LIF) often differentiate spontaneously and haphazardly, producing a mixture of differentiated cell types. More recently, ES cell lines have been produced that are able to grow in feeder free culture and under defined conditions.

The factors that influence stem cell self-renewal may either be stimulatory or inhibitory and may function extracellularly or intracellulary. In the case of the secreted factor LIF, it is known that its extracellular receptor is gp 130, and that activation of this protein is sufficient for inhibiting murine ES cell differentiation. Within the cell, a crucial downstream effector of gp130 is the signal trahsducer and activator of transcription-3 (STAT-3). Another molecule which is particularly important in maintaining stem cell pluripotency is the transcription factor Oct-4, which when downregulated artificially leads to the loss of the pluripotent state in ES cells or mice. Other signalling molecules that naturally inhibit ES cell self renewal, such as the mitogen-activated protein kinases, have also been elucidated. A major goal of stem cell research will be the discovery of natural and synthetic factors, drugs, polypeptides, genes, oligonucleotides, tissue culture media and conditions, specific conditioned media, feeder cells, and other stimuli that have the effect of promoting the expansion ahd retaining the differentiation potential of various types of stem cell This includes adult stem cells, which at present have not been expanded appreciable in cell culture.

The second great challenge of stem cell research is tq direct the differentiation of stem cells to particular cell types which are functional, can replace cells lost in various disease states, and result in a positive clinical outcome. Coaxing stem cells to begin differentiating is actually a fairly straightforward process. For instance, ES cells removed from feeder cultures and grown to confluence on an adherent substrate will begin to differentiate spontaneously. Similarly, ES cells removed from feeder cultures and grown on a non-adherent substrate will form embryoid bodies - clusters of undifferentiated and partially differentiated cells from all three germ layers. These cells can be subsequently dissociated and plated in monolayer culture, and exposed tp factors that promote directed differentiation. Cultures exposed to these factors are more likely to be populated by fewer types of differentiated cell, compared to embryoid bodies or untreated cultures of differentiating cells which comprise mixtures of many different cell types. Nevertheless, few if any conditions have-been devised thus far that produce substantially pure cultures of differenced cells. In addition it is not clear if any of the protocols devised for stem cell differentiation yield cells that are suitable for cell replacement therapy - it may be that the cells have not terminally differentiated into the precise phenotype required, or that the differentiated cells are no longer viable in vivo.

The factors that have been used to induce directed differentiation bf stem cells include, retinoic acid, epidermal growth factor (EGF), bone morphogenic proteins (BMPs), basic fibroblast growth factor (bFGF), activin-A, transforming growth factor beta-1 (TFG β-1), hepatocyte growth factor, nerve growth factor, sonic hedgehog (SHH), interleukin-3 (IL-3), interleukin-6 (IL-6), granulocyte macrophage colony stimulating factor (GM-CSF), erythropoietin, vitamin D3, dexamethasone, β-mercaptoethanol, butylated hydroxyanisole, 5-azacytidine, DMSO, insulin, thyroid hormone (T3), LIF, foetal calf serum, vascular endothelial growth factor (VEGF), steel factor, variations in oxygen concentration, ascorbic acid, β-glycerophosphate, nicotinamide, platelet derived growth factor (PDGF), cAMP, various cell adhesion molecules and substrates, and others. In addition to these defined factors, it is likely that undefined extracts, such as conditioned media, human and animal tissue homogenates, or plant extracts can be used to direct stem cell differentiation. Progressive fractionation of these undefined extracts may yield active fractions or even pure components with high potency. These factors can be added to the growth medium used in a particular experiment, either alone, or in combination, or in a defined order which is crucial to the experimental result.

Many systems that have been devised for the differentiation of stem cells in vitro are complex multi-stage procedures, in which the precise nature of the various steps, as well as the chronology of the various steps, are important. For instance, Lee et al (2000, Nature Biotechnology, vol. 18, p 675-679) used a five stage protocol to derive dopaminergic neurons from mouse ES cells: 1) undifferentiated ES cells were expanded on gelatin-coated tissue culture surface in ES cell medium in the presence of LIF ; 2) embryoid bodies were generated in suspension cultures for 4 days in ES cell medium; 3) nestin-positive cells were selected from embryoid bodies in ITSFn medium for 8 days after plating on tissue culture surface; 4) nestin-positive cells were expanded for 6 days in N2 medium containing bFGF/laminin; 5) finally the expanded neuronal precursor cells were induced to differentiate by withdrawing bFGF from N2 medium containing laminin.

In a second example of serial cell culture, Bonner-Weir et al. [Proc. Natl. Acad. Sci. (2000) 97: 7999-8004]- derived insulin producing cells from human pancreatic ductal cells by: 1) selecting ductal cells over islet cells by selective adhesion on a solid surface in the presence of serum for 2-4 days; 2) subsequently withdrawing serum and adding keratinocyte growth factor to select for ductal epithelial cells over fibroblasts for 5-10 days; and 3) overlaying the cells with the extracellular matrix preparation Matrigel' for 3-6 weeks.

In a further example of serial cell culture, Lumelsky et al. [Science (2001) 292: 1389-1394] derived insulin secreting cells by directed differentiation of mouse embryonic stem (ES) cells by: 1) expanding ES cells in the presence of LIF for 2-3 days 2) generating embryoid bodies in the absence of LIF over 4 days; 3) selecting nestin-positive cells using ITSFn medium for 6-7 days; 4) expanding pancreatic endocrine precursors in N2 medium containing B27 media supplement and bFGF for 6 days; and 5) ihducing differentiation to insulin secreting cells by withdrawing bFGF and adding nicotinamide.

However it is not only the sequence and duration of the various steps or the series of addition of different factors that is important in the determination of cell differentiation. As embryonic development is regulated by the action of gradients of signalling factors that impart positional information, it is to be expected that the concentration of a single signalling factor, and also the relative concentrations of two or more factors, will be important in specifying the fate of a cell population in vitro and in vivo. Factor concentrations vary during development and stem cells respond differently to different concentrations of the same molecule, For instance, stem cells isolated from the CNS of late stage embryos respond differently to different concentrations of EGF: low concentrations of EGF result in a signal to proliferate, while higher concentration of EGF result in proliferation and differentiation to astrocytes.

Many of the factors that have been found to influence self-renewal and differentiation of stem cells in vitro are naturally-occurring molecules, This is to be expected, as differentiation is induced and controlled by signalling molecules and preceptors that act along signal transduction pathways. However, by the same token, it is likely that many synthetic compounds will have an effect on stem cell differentiation. Such synthetic compounds that have high probability of interacting with cellular targets within signalling and signal transduction pathways (so called drugable targets) are routinely synthesised, for instance for drug screening by pharmaceutical companies. Once known, these compounds can be used to direct the differentiation of stem cells ex vivo, or can be administered in vivo in which case they would act on resident stem cells in the target orgah of a patient.

### Common variables in tissue culture

In developing conditions for the successful culture of a particular cell type, or in order to achieve or modulate a cellular process, it is often important to consider a variety of factors.

One important factor is the decision of whether to propagate the cells in suspension or as a monolayer attached to a substrate. Most cells prefer to adhere to a substrate although some, including transformed cells, haematopoietic cells, and cells from ascites, dan be propagated in suspension.

Assuming the culture is of adherent cells, an important factor is the choice of adhesion substrate. Most laboratories use disposable plastics as substrates for tissue culture. The plastics that have been used include polystyrene (the most common type), polyethylehe, polycarbonate, Perspex, PVC, Teflon, cellophane and cellulose acetate. It is likely that any plastic can be used, but many of these will need to be treated to make them wettable and suitable for cell attachment. Furthermore it is very likely that any suitably prepared solid substrate can be used to provide a support for cells, and the substrates that have been used to date include glass (e.g. alum-borosilicate and soda-lime glasses), rubber, synthetic fibres, polymerised dextrans, metal (e.g. stainless steel and titanium) and others.

Some cell types, such as bronchial epithelium, vascular endothelium, skeletal muscle and neurons require the growth substrate to be coated with biological products, usually extracellular matrix materials such as fibronectin, collagen, laminin, polylysine or others. The growth substrate and the method of application (wet or dry-coating, or gelling) can have an effect on cellular processes such as the growth and differentiation characteristics of cells, and these must be determined empirically as discussed above.

Probably the most obviously important of the variables in cell culture is the choice of culture medium and supplements such as serum. These provide an aqueous compartment for cell growth, complete with nutrients and various factors, some of which have been listed above, others of which are poorly defined. Some of these factors are essential for adhesion, others for conveying information (e.g. hormones, mitogens, cytokines) and others as detoxificants. Commonly used media include RPMI 1640, MEM/Hank's salts, MEM/Earle's salts, F12, DMEM/F12, L15, MCDB 153, and others. The various media can differ widely in their constituent - some of the common differences include sodium bicarbonate concentration, concentration of divalent ions such as Ca and Mg, buffer composition, antibiotics, trace elements, nucleosides, polypeptides, synthetic compounds, drugs, etc. It is well known that different media- are selective, meaning they promote the growth of only some cell types. Media supplements such as serum, pituitary brain and other extracts, are often essential for the growth of cells in culture, and in addition are frequently responsible for determining the phenotype of cells-in culture, i.e. they are capable of determining cell survival or directing differentiation. The role of supplements in cell processes such as differentiation is complex and depends on their concentration, the time point at which they are added to the culture, the cell type and medium used. The undefined nature of these supplements, and their potential to affect the cell phenotype, have motivated the development of serum-free media. As with all media, their development has come about largely by trial and error as has been discussed above.

The gas phase of the tissue culture is also important and its composition and volume which should be used can depend on the type of medium used, the amount of buffering required, whether the culture vessel is open or sealed, and Whether a particular cellular process needs to be modulated. Common variables include concentration of carbon dioxide and oxygen.

other conditions important to tissue culture include the choice of culture vessel, amount of headspace, inoculation density, temperature, frequency of media changes, treatment with enzymes, rate and mode of agitation or stirring.

Varying the cell culture conditions is therefore a method of achieving a desired cellular process. One aspect recognises that variation of the cell culture conditions in a serial manner can be a highly effective method for achieving a cellular effect. In various applications, for instance in studies of cell differentiation, it will often be the case that a specific series of different tissue culture conditions are required to effect a cellular process. The different conditions may include additions or withdrawals to/from the media or the change of media at specific time points. Such a set of conditions, examples of which are given below, are commonly developed by trial end error as has been discussed above.

### Formation of cell units

An important aspect is that groups of cells (cell colonies) can be grown in cell culture under various conditions and that the colony can largely maintain its integrity undervarious conditions, when disturbed, and when mixed with other colonies. Such groups or colonies are referred to herein as cell units. Formation of cell units may be achieved, by way of illustration, by growing cells as adherent cultures on solid substrates such as carriers. If cell proliferation occurs after seeding on the carriers, the daughter cells will attach on the same carrier and form part of the same colony. In general, live adherent cells do not readily dissociate from their growth substrate, and so the integrity of the cell colony persists despite any mechanical manipulation of the carrier, agitation of the culture medium, or transfer into another tissue culture system. Similarly, if at any time multiple carriers are placed in the same vessel (e.g. beads are pooled) then there will be no substantial transfer of cells from one bead to another.

One advantage of growing cells in units or colonies is that if a unit is placed serially in a set of different tissue culture media, then all the cells comprising the colony will have been exposed to the same series of culture conditions, in the same order and for the same period of time.

Another of the advantages of this method- is that tissue culture can be miniaturised: relatively few cells are required to colonise a microcarrier bead (see below) compared to even the smallest tissue culture flask.

Growing cells in units that are not necessarily themselves adherent to the tissue culture vessel has the further advantage that individual colonies can be removed at will and transferred to a different culture vessel. This is particularly important as it allows for the transfer of differentiated cells comprising progenitors to microtitre plates suitable for drug screening. Thus the progenitors can be prepared in bulk - such as by a method previously determined using the technique disclosed in WO2004/031369, and then the same cell units transferred essentially by liquid transfer into the HTS platform, This (i) greatly facilitates the HTS procedure, (ii) maintains the 3D (multi-) cellular organisation of units which may be integral to further differentiation, (iii) and obviates any dissociation of cellular organisation which may in itself cause cellular differentiation.

A further advantage of growing cell units formed on carriers is that cell culture pain be scaled up. Growth of stem cells on carriers offers a way of scaling up production to provide enough material for high throughput screening. Scale up of such cell cultures may require at least 1 g (dry weight) of microcarrier - such as 10g, 50g, or more. Another important advantage of forming cell units on solid substrates is that the substrate - and therefore the attached cells by reason of association can be labeled by various-means.

Glass spheres of 3mm and 5mm have been widely used as cell adhesion substrates, particularly in glass bead bioreactors (e.g. such as manufactured by Meredos Gmbh) used for the scale-up of cell cultures. These beads are typically used in packed beds rather than batch culture, to avoid mechanical damage to the adhereht cells. Though such substrates are suitable for the purposes of this invention, other carriers described below may be even more suitable.

In particular when cells are grown on smaller carriers they can be treated as a suspension culture. Importantly, a common method of growing cells on small carriers is referred to as microcarrier cell culture (see 'Microcarrier cell culture, Principles and Methods', Editidn AA, available from Amersham Biosciences (18-1140-62); Micorcarrier cultures are used commercially for antibody and interferon production in fermenters of up to 4000 litres. A variety of microcarriers is available, ranging in shape and size and made of different materials. Microcarrier beads made of polystyrene (Biosilon, Nunc), glass (Bioglass, Solohill Eng), collagen (Biospheres, Solohill Eng), DEAE sephadex (Cytodex-1, Pharmacia), dextran (Dormacell, Pfeifer & Langen), cellulose (DE-53, Whatman), gelatin (Gelibead, Hazelton Lab), and DEAE dextran (Microdex, Dextran Prod.) amongst others are commercially available. These carriers are well characterised in terms of the specific gravity of the beads, the diameter and the surface area available for cell growth. In addition a number of porous (micro) carriers are available with greatly increased surface area for cell growth. A further characteristic of these porous carriers is that they are suitable for growth of both anchorage dependent cells, as well as for suspension cells which are carried by entrapment in the network of open, interconnecting pores. Porous carriers are available in materials such as gelatin (Cultispher G, Percell), cellulose (Cytocell, Pharmacia), polyethylyne (Cytoline 1 and 2, Pharmacia), silicone rubber (Immobasil, Ashby Scientific), collagen (Microsphere, Cellex Biosciences), and glass (Siran, Schott Glassware). These carriers are variously suited to stirred, fluidised or fixed bed culture systems.

As the physical properties of carriers are well known it is easy to calculate the number of carriers used in an experiment. Some of the carriers described and many besides are available as dried products which can be accurately weighed, and subsequently prepared by swelling in liquid medium. In addition the number of cells used to inoculate a microcarrier culture can be worked out and varied. For instance, a culture of Cytodex 3 (2 g/litre) inoculated at 6 cells per bead will give a culture containing 8 million microcarriers, on which 48 million cells/litre are grown at a density of 5x10⁴ cells/cm².

If required, harvesting of cells grown on microcarriers, or liberation of labels from microcarriers (see below), can be achieved by enzymatic detachment of cells, and/or by digestion of the carrier where applicable: gelatin carriers can be solubilised by trypsin and/or EDTA, collagen carriers using collagenase and dextran carriers using dextranase.

In addition to solid or porous microcarriers, cells may be grouped by immurement, i.e. confined within a medium permeable barrier. Membrane culture systems have been developed where a permeable dialysis membrane retains a group of cells, but allows the culture medium and its constituents to exchange freely with the inner and outer compartments. Cell culture in hollow fibre cartridges has also been developed, and a multitude of fibres-and even turn-key systems are commercially available (e.g. from Amicon, Cellex Biosciences). Cell encapsulation in semi-solid matrices has also been developed, where cells are immobilised by adsorption, covalent bonding, crosslinking on entrapment in a polymeric matrix. Materials that have been used include gelatin, polylysine, alginate and agarose. A typical protocol, is to mix 5% agarose at 40°C with a suspension of cells in their normal growth medium, to emulsify the mixture using an equal volume of paraffin oil and to cool in an ice bath, producing spheres of 80-200µm diameter. These spheres can be separated from the oil and transferred to medium in a tissue culture vessel.

Cell entrapment is a simple method for the immobilisation of groups of cells, akin to the use of microcarriers or porous substrates. A simple technique is to enmesh cells in cellulose fibres such as DEAE, TLC, QAE, TEAE (all available from Sigma). Other more sophisticated devices are ceramic cartridges which are suitable for suspension cells, as in the Opticel culture system (Cellex Biosciences).

One skilled in the art will envisage, in addition to the above methods of creating cell units, other methods of creating groupings of cells including forming 3D cultures of cells such as neural spheres or embryoid bodies, or using tissues and indeed whole organisms such as Drosophilia or C. elegans.

Cell units, or the substrates of which they are comprised, can be associated with a particular factor including, but without limitation, proteins, nucleic acids or other chemicals such as drugs. Pre-conditioning of substrates can be achieved in many ways, for instance simply by incubating the substrate with the factor of interest or by attaching the factor covalently or non-covalently to the substrate. Soluble factors can be incorporated into dry materials by impregnation. This technique relies on the rapid ingress of liquid, carrying soluble factors, into dry porous material that concomitantly becomes swollen and ready for use. Solid factors can be incorporated for example by mixing the factor in fibrinogen with thrombin solution, at which point a fibrin clot containing the factor is formed. Multiple other ways can be envisaged of associating factor(s) with a cell group, in addition to impregnating, entrapping or encapsulating the factor together with cells.

A method for associating a cell group with a number of different factors is to pre-form cocktails of factors which are subsequently associated with a particular cell group. A second method would be to serially condition cell groups in a number of factors. Using dry formulations of cell group growth substrates as an example, this method would involve firstly partially swelling the substrate in a solution containing a first factor and subsequently further swelling the same in a solution containing a second factor, resulting in a substrate to which both factors have become associated. By devising a systematic protocol of associating cell groups with different combinations of different factors it will be possible to sample the effect on the cell group of any combination of a set of factors.

Regardless of the method used to condition cell units with factors, the factors are taken up by cells that comprise that cell unit. Factors leaking into the growth medium are diluted to such an extent that their concentration falls below physiologically relevant limits and they have no effect on any additional cell group to which they are exposed. The diffusion of the factor out of e.g. the substrate forming port of the cell unit is governed by parameters such as the nature and dimensions of the material, the mean pore diameter, and the molecular weight and concentration of the factor. To calibrate the process if necessary, factor release can be measured by physical assays such as HPLC analysis or release of labelled factor into the medium, or by biological assays such as the dorsal root ganglion outgrowth bioassay for neurotrophic factors.

### Combinatorial serial culture of cells

The invention further addresses the problem that cell culture technique involving a plurality of steps and agents are difficult if not impossible to determine by conventional experimentation, which in the -prior art has involved trial and error. Empirical determination of tissue culture condition in complex, multi-stage procedures is not feasible in practice. Advantageously, the culture conditions required to differentiate a cell type may first be identified using the methods described herein below.

### (a) Split-pool cell culture

Split-pool culturing allows cells to be subjected to a series of culture conditions, and exposed to a series of agents in culture media, in a systematic and highly productive manner and is described in detail in WO2004/031369.

The first step in split-pool cell culture is to form cell units (particularly microscopic cell units) as each cell unit constitutes an easily handled unit that can be exposed to a variety of cell culture conditions. For simplicity, in this discussion we will assume that cell groupings are produced by growing cells in microcarrier culture, and the terms cell unit, cell group, colony and bead are used interchangeably. However, the methods described are equally applicable to any cell unit. A particularly efficient method for sampling a large number of cell culture conditions is referred to as Combinatorial Cell Culture or split-pool cell culture and in one embodiment involves the serial subdividing and combining of groups pf cell units in order tq sample multiple combinations of cell culture conditions. In one aspect of the invention the method operates by taking an initial starter culture (or different starter cultures) of cell units divided into X₁ number of aliquots each containing multiple beads (groups/colonies/carriers) which are grown separately under different culture conditions. Following cell culture for a given time, the cell units can be pooled by combining and mixing the beads from the different aliquots. This pool cah be split again into X₂ number of aliquots, each of which is cultured under different conditions for a period of time, and subsequently also pooled. This iterative procedure of splitting, culturing and pooling (or pooling, splitting and culturing; depending on where one enters the cycle) cell units allows systematic sampling of many different combinations of cell culture conditions. The complexity of the experiment, or in other words the number of different combinations of cell culture conditions tested, is equal to the product of the number of different conditions (X₁ x X₂x ...Xₙ) sampled at each round. Note that the step of pooling all the cell units prior to a subsequent split can be optional - a step in which a limited number of cell units are pooled can have the same effect. The disclosure therefore embodies a number of related methods of systematically sampling multiple combinations of cell culture conditions where groups of cell units are handled in bulk.

Regardless of the precise manner in which a diversity of cell culture conditions is sampled by this means the procedure is efficient because multiple cell units can share a single vessel, where they are cultured under identical conditions, and it can be carried out using only a few culture vessels at any one time (the number of culture vessels in use is equal to the number of split samples). In many respects the principle of this procedure resembles that of split synthesis of large chemical libraries (known as combinatorial chemistry), which samples all possible combinations of linkage between chemical building block groups (see for example: Combinatorial Chemistry, Oxford University Press (2000), Hicham Fenniri (Editor)). Split-pool cell culture- can be repeated over any number of rounds, and any number of conditions can be sampled at each round. So long as the number of cell units (or colonised beads in this example) is greater than or equal to the number of different conditions sampled over all rounds, and assuming that the splitting of cell units occurs totally randomly, it is expected that there-will be at least one cell unit that has been cultured according to each of the various combinations of culture conditions sampled by the experiment. This procedure can be used to sample growth or differentiation conditions for any cell type, or the efficiehcy of biomolecule production (e.g. production of erythropoeitin or interferon) by any cell type. Because the procedure is iterative, it is Ideally suited to testing multistep tissue culture protocols - for instance those described above in connection with stem cell differentiation. The variables which can be sampled using this technique include cell type, cell grouping (e.g. microcarrier culture, cell encapsulation, whole organism), growth substrate (e.g. fibronectin bn microcarrier), duration of cell culture round, temperature, different culture media (including different concentrations of constituents), growth factors, conditioned media, co-culture with various cell types (e.g., feeder cells), animal or plant extracts, drugs, other synthetic chemicals, infection with viruses (incl. transgenic viruses), addition of transgenes, addition of antisense or anti-gene molecules (e.g. RNAi, triple helix), sensory inputs (in the case of organisms), electrical, light, or red-ox stimuli and others.

In one embodiment, the culture conditions required to differentiate the first cell type pre first identified in a method comprising the steps of: (a) providing a first set of groups of cell units each comprising one or more cells, and exposing said groups to desired culture conditions; (b) subdividing one or more of said groups to create a further set of groups of cell units; (c) exposing said further groups to further desired culture conditions; (d) optionally, repeating steps (b)-(c); and (e) assessing the effect on a given cell Unit of the culture conditions to which it has been exposed.

In another embodiment, the culture conditions required to partially of fully differentiate the first cell type are first identified in a method comprising the steps of:
(a) providing a first set of groups of cell units each comprising one or more cells, and exposing said groups to desired culture conditions; (b) pooling two or more of said groups to form at least one second pool; (c) subdividing the second pool to create a further set of groups of cell units; (d) exposing said further groups to desired culture conditions; (e) optionally, repeating steps (b) - (d); and (f) assessing the effect on a given cell unit of the culture conditions to which it has been-exposed.

Suitably, cell units that are partially differentiated are isolated and used in the methods described herein.

Suitably, cell units are labelled and the label(s) reflect(s) the culture conditions to which the cell unit has been exposed. The label may be spatially encoded. The label may be selected from the group consisting of a virus, an oligonucleotide, a peptide, a fluorescent compound, a secondary amine, a halocarbon, a mixture of stable isotopes, a bar code, an optical tag, a bead, a quantum dot and a radiofrequency encoding tag or combinations comprising at least two of these labels. Two or more labels may be selected and used in combination to label a cell unit.

Suitably, the cells are cultured in cell units, each cell unit comprising one or more cells. The cell units may be single cells. Each cell unit may comprise one or more cells adherent to or bounded by a solid substrate. The solid substrate may be a microcarrier or bead. The solid substrate may even be a well or medium-permeable barrier.

In one embodiment, the culture conditions are media to which the cell is exposed. Suitably, the media contain one or more specific agents which influence a cellular process.

The cell culture conditions may comprise culturing at one or more specific temperatures or partial pressures of oxygen or carbon dioxide. The cell culture conditions may comprise culturing on one or more specific substrates.

### (b) Split-split cell culture

The purpose of performing split-pool processes on cell units is to systematically expose these to a pre-defined combination of conditions. The person skilled in the art will conceive of many different means of achieving this outcome. In addition to split-pool processes and variations thereof, it is worthwhile briefly discussing split-split processes. A split-split process involves subdividing a group of cell units at least twice, without intervening pooling of cell units. If split-split processes are used over a large number of rounds, the number of separate samples that are generated increases exponentially. In this case it is important to employ some level of automation, for example the use of a robotic platform and sophisticated sample tracking systems. The advantage of split-split steps is that (since cell units are hot combined) it is possible to segregate lineages of the various cell units based on their cell culture history. Consequently split-split steps can be used to deduce if a particular cell culture condition is responsible for any given cellular process and therefore used to deduce the culture history of cell units (explained in detail under Determination of culture history of a cell unit').

### Predetermined protocols

The splitting and/or pooling of cell units may be accomplished totally randomly or may follow a predetermined protocol. Where cell units are split and/or pooled randomly, the segregation of a given cell unit into any group is not predetermined or prejudiced in any way. In order to result in a high probability that at least one cell unit has been exposed to each of the possible combinations of cell culture conditions, it is advantageous to employ a larger number of cell units than the total number of combinations of cell culture conditions that are being tested. Under certain circumstances it is therefore advantageous to split and/or pool cell units according to a predetermined protocol, the overall effect being that adventitious duplication or omissions of combinations are prevehted. Predetermined handling of cell units can be optionally planned in advance and logged on a spreadsheet or computer programme, and splitting and/or pooling operations executed using automated protocols, for instance robotics. Labelling of cell units (see below) can be by any of a number of means, for instance labelling by RFID, optical tagging or spatial encoding. Robotic devices capable of determining the-identity of a sample, and therefore partitioning the samples according to a predetermined protocol, have been described (see 'Combinatorial Chemistry, A practical Approach', Oxford University Press (2000), Ed H. Fenniri). Alternatively, standard laboratory liquid handling and/or tissue culture robotics (for example such as manufactured by: Beckman Coulter Inc, Fullerton, CA; The Automation Partnership, Royston, UK) is capable of spatially encoding the identity of multiple samples and of adding, removing or translocating these according to pre-programmed protocols.

### Analysis and/or separation of cell units

Following each round of cell culture, or after a defined number of rounds, the cell units can be studied to observe any given cellular process that may have been affected by the tissue culture conditions. The examples below are illustrative and not intended to limit the scope of the invention.

Following each round of cell culture, or after a defined number of rounds, the cell units can be assayed to determine Whether there are members displaying increased cell proliferation. This can be achieved by a variety of techniques, for instance by visual inspection of the cell units under a microscope, or by quantitating a marker product characteristic of the cell. This may be an endogenous marker such as a particular DNA sequence, or a cell protein which can be detected by a ligand or antibody. Alternatively an exogenous marker, such as green fluorescent protein (GFP), can be introduced into the cell units being assayed to provide a specific readout of (living) cells. Live cells can be visualised using a variety of vital stains, or conversely dead cells can be labelled using a variety of methods, for instance using propidium iodide. Furthermore the labelled cell units can be separated from unlabelled ones by a variety of techniques, both manual and automated, including affinity purification ('panning'), or by fluorescence activated cell sorting (FACS) or broadly similar techniques. Depending on the application it may be possible to use standard laboratory equipment, or it may be advantageous to use specialised ipstrumentation. For instance, certain analysis and sorting instruments (e.g. see Union Biometrica Inc., Somerville MA, USA) have flow cell diameters of up to one millimeter, which allows flow sorting of beads with diameters up to 500 microns. These instruments provide a reading of bead size and optical density as well as two fluorescent emission wavelengths from tags such as GFP, YFP or DS-red. Sorting speeds of 180,000 beads per hour and dispensing into multi-well plates or into a bulk receptor are possible.
Following each round of cell culture, or after a defined number of rounds, the cell units can be assayed to determine whether there are members displaying a particular genotype or phenotype. Genotype determination can be carried out using well known techniques such as the polymerase chain reaction (PCR), fluorescence in situ hybridisation (FISH), DNA sequencing, and others. Phenotype determination can be carried out by a variety of techniques, for instance by visual inspection of the cell units under a microscope, or by detecting a marker product characteristic of the cell. This may be an endogenous marker such as a particular DNA or RNA sequence, or a cell protein Which can be detected by a ligand, conversion of an enzyme substrate, or antibody that recognises a particular phenotypic marker (For instance see Appendix E of Stem Cells: Scientific Progress and Future Research Directions. Department of Health and Human Services. June 2001. A genetic marker may also be exogenous, i.e. one that has been introduced into the celt population, for example by transfection or viral transduction. Examples of exogenous markers -are the fluorescent proteins (e.g. GFP) or cell surface antigens-which are not normally expressed in a particular cell lineage or which are epitope-modified, or from a different species. A transgene or exogenous marker gene with associated transcriptional control elements can be expressed in a manner that reflects a pattern representative of an endogenous gene(s). This can be achieved by associating the gene with a minimal cell type-specific promoter, or by integrating the transgene into a particular locus (e.g. see European patent No. EP 0695351). The labelled cell units can be separated from unlabelled ones by a variety of techniques, both manual and automated, including affinity purification ('panning'), or by fluorescence activated cell sorting (FACS). Nishikawa et al (1998, Development vol 125, p1747-1757) used cell surface markers recognized by antibodies to follow the differentiation of totipotent murine ES cells. Using FACS they were able to identify and purify cells of the haematopoietic lineage at various stages in their differentiation.

An alternative or complementary technique for enriching cell units of a particular genotype or phenotype is to genetically select the desired groups. This can be achieved for instance by introducing a selectable marker into the cell units, and to assay for viability under selective conditions, for instance see Soria et al (2000, Diabetes vol 49, p1-6) who used such a system to select insulin secreting cells from differentiated ES cells. Li et al (1998, Curr Biol vol 8, p 971-974) identified neural progenitors by integrating the bifunctional selection marker/reporter βgeo (which provides for β-galactosidase activity and G418 resistance) into the Sox2 locus by homologous recombination in murine ES cells. Since one of the characteristics of neural progenitors is expression of Sox2, and therefore the integrated marker genes, these cells could be selected from non-neuronal lineages by addition of G418 after inducing differentiation using retinoic acid. Cell viability could be determined by inspection under a microscope, or by monitoring β-gal activity. Unlike phenotype-based selection approaches, which can be limited by the availability of ah appropriate ligand or antibody, genetic selection can be applied to any differentially expressed gene.

### Microcarriers

A variety of microcarriers are available, ranging in shape and size and made of different materials.

By way of example, the microcarrier may-be a porous microcarrier selected from the group consisting of Cytopore microcarrier (eg. a Cytopore 1 microcarrier or a Cytopore 2 microcarrier), a Cultispher microcarrier, a Cultispher-G microcarrier, a Cultispher-GL microcarrier and a Cultispher-S microcarrier, an Informatrix microcarrier, a Microsphere microcarrier, a Siran microcarrier, and a Microporous MC microcarrier.

By way of Further example, the microcarrier may be a solid microcarrier - such as a Cytodex microcarrier (eg. a Cytodex 1, Cytodex 2 or Cytodex 3 microcarrier) a Biosilon microcarrier, a Bioglass microcarrier, a FACT III microcarrier or a DE 52/53 microcarrier.

Microcarrier culture has significant advantages, including the scale-up of cultures, and also allows units of cells to be exposed to selected culture conditions as required in order to obtain the desired growth and/or differentiation conditions.

The surfaces of the microcarriers may be further modified by physical or chemical treatments, such as adsorption or covalent cross-linking of molecular entities with a desired charge or other desired characteristic.

### Cultispher microcarriers

CultiSpher is manufactured from pharmaceutical grade porcine gelatin via a process which yields a highly cross-linked gelatin matrix with high mechanical and -thermal stability. When used in cell cultures, cells can attach to both the external and the internal surfaces of the matrix. The increased surface area of the matrix together with the protection from stress afforded to the cells in the interior of the matrix results in enhanced cell production capabilities. An additional advantage of the product is that the matrix can be dissolved with proteolytic enzymes resulting in the harvesting of cells with almost 100% viability.

In one embodiment, the microcarrier is a Cultispher-G microcarrier. Cultispher-G has a particle diameter of 130-380 µm, a volume of 12-18 ml/g dry, a density of 1.04 g/ml with an overage pore diameter of 20 µm.

In order to prepare and use Cultispher-G microcarriers, reference can be made to *inter alia* Biotech. Bioeng. (2000) 68, 1 p59-70; Brit, J. Cancer. Suppl. XXVII, S-78-S82 (1996); and the manufacturer's website at www.percell.se.

### Cytopore 2 microcarrier

Cytopore microcarriers, are available from GE Heaithcare (previously Amersham) (www.microcarriers.com). Cytopore is made of 100% cellulose, which is non-toxic to the cells and biodegradable. It is positively charged, due to the N,N,-diethylaminoethyl groups. It has a very precise particle size distribution and a network structure, the ratio of surface area to particle material is more than 95 to 1. The network structure enables stained cells to be closely observed while they grow inside the microcarriers. The typical particle diameter is 200-280 µm and effective surface area is 1.1 m2/g dry. The relative density is 1.03 g/ml, the average diameter of pore openings is 30 µm and the volume is 40 ml/g dry. In order to prepare and use Cytopore microcarriers reference is made to *inter alia* Applied Microbiology and Biotechnology (1997) 47, 4 p352-7; Cytotechnology (1999) 30 p143-147; Chinese Journal of Biotechnology (1999) 15, 4 p239-44 and Acta Oto-Laryngologica (2002) 122, 5 p541-5.

Cytppore 2 has been optimised for anchorage-dependent cells requiring a charge density around 1.8 meq/g.

In some embodiments, the microcarrier is a porcine gelatin microcarrier.

In some embodiments, the microcarrier is made of 100% cellulose.

In one embodiment, differentiated cells may be obtained from stem cells *in vitro* by a method comprising the steps of: (a) growing stem cells adherent to microcarriers in a culture medium; (b) transferring the microcarriers from one culture medium to another; (c) optionally repeating step (b) as required; and (d) obtaining the differentiated cells attached to the microcarrier.

The stem cells or partially differentiated cells may be exposed to a potential modulator whilst still attached to said microcarrier. The disclosure thus provides a method for using these cells in HTS while still attached to the microcarrier, such as carrying out cell transfer steps by Using robotics. The differentiated cells may be isolated by enzymatic detachment from the microcarrier. The differentiated cells may be isolated by digestion of the microcarrier.

The methods of the invention may be practised using more than 50g dry weight of microcarrier.

Pluripotent stem cells may be grown *in vitro* by a method comprising the steps of: (a) seeding said cells on microcarriers; and (b) propagating the cells while attached to the carriers.

### Determination of the identity or cell culture history of a cell unit

When handling large numbers of cell units, their identity and/or cell culture history (for example the chronology and the exact nature of a series of culture conditions that any one group or unit may have been exposed to) ban become confused. For instance, the split-pool protocol of cell culture necessarily involves mixing cell units in each round, making it difficult to follow individual units. Determining the cell culture history of a cell unit in a mixture of cell units which have been subjected to multiple culture conditions is sometimes referred to as 'deconvolution' of the cell culture history. One method of doing this is to label cell units and it is therefore advantageous to label the cell units. Labelling may be performed at the beginning of an experiment, or during each round of an experiment and may involve a unique label (which may or may not be modified in the course of an experiment) or a series of labels which comprise a unique aggregate. Similarly, reading of the label(s) may take place during each round or simply at the end of the experiment. In one embodiment, unique labels such as RFID labels are read during each round, whereas labels added serially at each round are read at the end of an experiment.

Labelling of cell units may be achieved by a variety of means, for instance labelling either the cells themselves, or any material to which the cells are attached or otherwise associated with. Any of the chemical and non-chemical methods used to encode synthetic combinatorial libraries can be adapted for this purpose and some of these are described in Methods in Enzymology Vol 267 (1996), 'Combinatorial Chemistry', John N. Abelson (Editor); Combinatorial Chemistry, Oxford University Press (2000), Hicham Fenniri (Editor); K. Braeckmans et al., 'Scanning the code', Modern Drug Discovery (Feb. 2003); K. Braeckmans et al,, 'Encoding microcarriers: Present and Future Technologies'; Nature Reviews Drug Discovery, vol. 1, p. 447-456 (2002). Some examples of labelling methods follow.

One method of labelling cell units involves associating cell units with a tag that becomes sequentially modified as it is placed in different culture conditions. This may involve for instance the addition or subtraction of further units to the tag such that its stereochemistry, sequence or mass is altered; or the alteration of electronic memory as in read-write RF transponders (see below).

Another method of labelling cell units involves sequentially associating unique tags with the cell units whenever they are cultured under different conditions, such that subsequeht detection and identification of the tags provides for an unambiguous record of the chronology and identity of the cell culture conditions to which the cell unit has been exposed. Tags can be taken up by cells, or attached to the cell surface by absorption, or a suitable ligand or antibody, or conjugated to a cell-associated matrix such as a carrier by adsorption, colloidal forces or a variety of linkages such as covalent linkage or non-covalent linkage, e.g. biotin-streptavidin linkage. For instance, one simple tag that can be introduced to cells or attached to a matrix associated with cells is an oligonucleotide of defined length and/jor sequence.

Oligonucleotides may comprise any class of nucleic acid (e.g. RNA, DNA, PNA, linear, circular or viral) and may contain specific sequences for amplification. (e.g. primer sequences for PCR) or labels for detection (e.g. fluorophores or quenchers, or isotopic tags). The detection of these may be direct, for instance by sequencing the oligos or by hybridising them to complementary sequences (e.g. on an array or chip), or indirect as by monitoring an oligonucleotide-encoded gene product, or the interference of the nucleotide with a cellular activity (e.g. antisense inhibition of a particular gene). An advantageous method of amplifying nucleic acids is by rolling circle amplification (RCA; 2002, V. Demidov, Expert Rev. Mol. Diagn. 2(6), p.89-95) where nucleic acid tags can comprise RCA templates, elongation primers, or struts that aid the circularization of minicircle templates).

Any molecular or macromolecular tag can be used so long as it can be detected, including peptide tags, coloured or fluorescent compounds, secondary amines, halocarbons, mixtures of stable isotopes etc. Tags may attach to cell units directly or via an intermediary, for instance an antibody raised against a component of the cell unit, or via an interacting pair such as biotin-steptavidin. In addition tags can be protected against degradation by the components of the cell culture, for example by chemical or other modification or by encapsulation. Encapsulation of tags can take place in many different media, for example in beads many types of which are available from suppliers such as Bangs Laboratories Inc. (Fishers IN, USA), and encapsulation may be used to standardise tag dosage in addition to providing components for tag amplification and/or detection (for example by providing PCR primers for use with a DNA tag). One method of labelling cell units employs fluorescent beads such as those manufactured by Luminex Corporation (Austin, TX, USA). The Luminex system comprises polystyrene beads which may or may not be externally derivatised (e.g. with avidin or anibody) and are internally dyed with differing ratios of two spectrally distinct fluorophores, and a reader which is capable of characterising the spectral signature of each bead. A further method employs beads such as those manufactured by Bangs Laboratories Inc. (Fishers IN, USA). The Bangs system comprises bead sets which can be distinguished based on differing sizes (e.g. bead sets of 4.4µm and 5.5µm diameter). Beads within each set can be furthermore distinguished from each other based on differing fluorescence intensity owing to differential loading with a single fluorescent dye. It is possible to use many different dyes with different absorption or emission characteristics, which can be internally loaded or attached externally to carriers by a multiplicity of means. It is furthermore possible to use 'quantum dots' to obtain a very high number of different fluorescent labels which can be read conveniently.

Cell growth substrates such as those described in connection with forming cell units can be derivatised or coated with substances that facilitate tagging and do not interfere with cell growth. One method of derivatising carriers is to modify them covalently or non-covalently with biotin, to which a tag can be attached via streptavidin or avidin. In general it will be important to use a tag that will not itself induce a cellular effect (i.e. an inert tag), and that can be distinguished from molecules present in cell units or the culture media, and that can be attached to its target and subsequently detected in the background of such molecules. To facilitate detection, it may be advantageous to selectively elute tags from cell units or to strip off the cells from cell units using selective conditions. More complicated molecular tagging strategies can also be envisaged, including the strategy of 'binary encoding' where information is recorded by a set of binary codes assigned to a set of molecular tags and their mixtures.

Detection of tags can be accomplished by a variety of methods familiar to those skilled in the art. Methods include mass spectrometry, nuclear magnetic resonance, sequencing, hybridisation, antigen detection, electrophoresis, spectroscopy, microscopy, image analysis, fluorescence detection, etc.

Of particular interest are labelling or encoding strategies in which labelling is carried out only once or where labelling and/or detection are non-physical and therefore non-invasive. Radiofrequency Identification (RFID) is-an example of a system exhibiting these properties. RFID employs transponders (RF tags), antennae and readers. An RF tag is a small electronic circuit, usually encased in glass or plastic, which in its -simplest form provides access to a unique identification code that may be 'read', without contact or line of sight, by suitable electronics. Tags may also store information generated by the user, agaih without contact or line of sight. A 'reader' is an electronic unit that transfers information to and from one or more tags (it should be noted that the term reader is used interchangeably to mean both a read only and read/write unit). The size and features of a reader may vary considerably, and it may operate in isolation, or be connected to a remote computer system. An antenna is used to transmit information from a reader to a tag, and to receive information sent by an RF tag. The size and format of an antenna will reflect the specific application, and may range from a small circular coil to large planar structures. An RFID system may operate in isolation, or be connected to a remote computer for more comprehensive interpretation and manipulation of identification and associated data derived from a tag. One RFID strategy used in combinatorial chemistry is described in Nicolaou et al (1995, Angew Chem Intl Ed Engl, vol. 34, p. 2289) and comprises:
(i) a porous enclosure containing a synthesis substrate and the semiconductor tag; (ii) the solid phase synthesis resin; (iii) a glass- encased Single or Multiple Addressable Radiofrequency Tag semiconductor unit capable of receiving, storing and emitting radiofrequency signals. A similar- device could be adapted to growing and following cell units simply by replacing the solid phase synthesis resin with tissue culture microcarriers or suitable cell units. More variations of this can be envisaged including but not limited to (coated or uncoated) RF tags on which cells are grown directly, or RF tags implanted into cell units or organisms.

Thus tags do not necessarily have to be distinguished by their chemical or molecular structure in the first instance. Multiple variations of the non-chemical tagging strategy can be devised to determine the identity of a given cell unit in a mixture or of deducing the identity of the different cell units that comprise a mixture. For instance optical or visual methods of tagging have been described where different shaped objects, graphically encoded objects or different colours denote the identity of a sample (for example see 1998, Guiles et al, Angew. Chem. Intl Ed Engl, vol. 37, p926; Luminex Corp, Austin TX, USA; BD Biosciences; Memobead Technologies, Ghent, Belgium), or where a pattern or bar code is etched onto a substrate such as a ceramic bar or nanowire and recognised using pattern recognition technology (for example see 1997, Xiao et al, Angew. Chem. Intl Ed Engl, vol 36, p780; SmartBead Technologies, Babraharn, UK; Oxonica Ltd, Kidlington, UK).

A further method of tracking or labelling cell units is to encode their identity spatially, i.e. by their position in space. In this method different cell units are segregated in defined relative positions, and these positions denote or encode the identity of the units. For instance, cell units may be cultured in an array, whereby the identity and/or culture history of each unit is known and is associated to a particular position in the array. In their simplest forms such arrays can comprise collection of tissue culture flasks, wells of a multi-well plate, or locations on a glass slide or other surface. Examples of positional encoding strategies can be found in Geysen et al. (1984, Proc Natl Acad Sci USA vol. 81, p. 3998-4002), Fodor et al. (1991, Science vol. 251, p. 767-773), Ziauddin and Sabatini (2001, Nature, Vol. 411, p. 107-110), and Wu et al. (2002, Trends Cell Biol. Vol. 12(10), p.485-8).

It will be apparent that it is not necessary to label all of the cell units in order to be able to deduce information about the outcomes resultant from a combination of cell culture protocols. Thus without labelling of cell units it would still be possible to assay large combinations of cell culture conditions according to the invention, and to determine whether one or more of these was capable of resulting in a particular cellular effect. However, in one embodiment, cell units are labelled. Labelling of a cell unit allows the derivation of useful information from the experiment regarding the outcome of the particular conditions sampled by the labelled cell unit, as opposed to all the cell units. Alternatively it is sometimes advantageous to label one or a few group(s) of cell units which have all been exposed to a certain culture protocol, for instance a group of cell units which have been segregated into the same medium during a particular split or pool step. It will also be apparent that labelling certain cell units allows one to infer the identity of other (perhaps unlabelled) cell units.

Similarly, it will be clear that performing cell culture experiments in which various conditions are omitted can give information regarding the utility of those conditions with respect to a particular experimental outcome. It would therefore be possible to evaluate each of the conditions sampled by repeating the experiment a number of times, each time omitting a different set of conditions.

Split-split cell culture steps can also be used to determine the effect of a particular set of conditions on experimental outcome. In effect split-split steps result in the formation of particular lineages of cell units which have each been exposed to a unique cell culture conditions at the time of branching. By studying the different lineages it is possible to determine the utility of the tissue culture conditions studied at the branching point, with respect to a particutar experimental outcome.

### Haematopoietic cell

In a further aspect, there is provided a method for producing a haematopoietic cell from a stem cell - such as an embryonic stem cell - *in vitro* comprising exposing said stem cell to one or more, preferably, two or more, reaction conditions, wherein said reaction conditions comprise incubating said stem cell with: (a) retinoic acid, dimethylsulphoxide (DMSO) and/or stem cell factor (SCF); and (b) insulin, stem cell factor (SCF), TGF beta 1, BMP2, BMP4 and/or TPO; and (c) IL-3, IL-6, TPO, EPO and/or M-CSF.

The stem cell may be seeded on a microcarrier - such as a gelatin microcarrier.

Typically, the stem cells are contained a medium - such as an IMDM basal (medium or a Streamline Haematopoietic Expansion Medium.

Suitably, in step (a) retinoic acid or dimethylsulphoxide (DMSO) or stem cell factor (SCF) are used; suitably in step- (b) insulin alone is used. Suitably, in step (b) SCF, TGF beta 1, BMP2 and TPO is used. Suitably, in step (c) IL-3 and IL-6 are used and optionally TPO, EPO and/or M-CSF are used.

Typically, step (a) is performed on day 1. Typically, step (b) is performed on day 4. Typically, step (c) is performed on day 6.

In one specific embodiment, the conditions are to incubate the stem cell with retinoic acid, dimethylsulphoxide (DMSO), or stem cell factor (SCF); then incubate the stem cell with insulin, stem cell factor (SCF), TGF beta 1, BMP2 or 4 and TPO or incubate the stem cell with insulin alone and/or a combination of SCF, TGF beta 1, BMP2 and TPO; and then incubate the stem cell with IL-3, IL-6, and optionally TPO, EPO and/or M-CSF.

In another specific embodiment, the conditions are on day 1 incubate the stem cell with retinoic acid or dimethylsulphoxide (DMSO), or stem cell factor (SCF); on day 4 incubate the stem cell with stem cell factor (SCF), TGF beta 1, BMP2, BMP4 and TPO or incubate the stem cell with insulin alone and/or a combination of SCF, TGF beta 1, BMP2 and TPO; on day 6 incubate the stem cell with IL-3, IL-6, and optionally TPO, EPO and/or M-CSF.

### Further aspects

Further aspects of this invention are presented in the accompanying paragraphs:
1. A method for identifying a potential modulator of a cell signalling pathway, comprising the steps bf:
   (a) providing a cell of a first cell type wherein said first bell type may be differentiated to a second cell type by sequentially exposing said first cell type to one or more reaction conditions;
   (b) adding to or replacing at least one of said one or more reaction conditions with exposure to one or more different reaction conditions comprising said potential modulator; and
   (c) monitoring the differentiation of the first cell type tb determine formation of the second cell type.
2. A method for identifying a potential modulator of a cell signalling pathway, comprising the steps of:
   (a) providing a cell of a first cell type, wherein said first cell type is obtained from an embryo or foetus and may be differentiated to a second cell type;
   (b) optionally amplifying the said first cell type;
   (c) further differentiating the said first cell type by sequentially exposing said first cell type to one or more reaction conditions;
   (d) exposing the first cell type to one or more different reaction conditions comprising said potential modulator; and
   (e) monitoring the differentiation of the first cell type cell to determine formation of the second cell type.
3. A method according to paragraph 1 or 2, wherein the reaction conditions are the culture conditions to which cells are exposed.
4. A method according to paragraph 2 or 3, wherein the first cell type is a cell which has been arrested along a differentiation pathway between a stem cell and a differentiated cell type.
5. A method according to paragraph 1 or 3 wherein the cell type is a primary cell, cell line or tumour derived cell line.
6. A method according to paragraph 4 or 5 wherein the tissue of origin of the cell type is selected from a group consisting of brain, heart, liver, lung, kidney, skin, hair, eye, tooth, pancreas, muscle, bone and vasculature.
7. A method according to any preceding paragraph, wherein the potential modulator is an inhibitor of a cell signalling pathway.
8. A method according to paragraphs 1 to 6, wherein the potential modulator is a promoter of a cell signalling pathway.
9. A method according to paragraphs 3 to 8 wherein the culture conditions required to differentiate-a cell type are first identified in a method comprising the steps of:
   (a) providing a first set of groups of cell units each comprising one or more cells, and exposing-said groups to desired culture conditions;
   (b) subdividing one or more of said groups to create a further set of groups of cell units;
   (c) exposing said further groups to further desired culture conditions;
   (d) optionally, repeating steps (b)-(c) iteratively, as required; and
   (e) assessing the effect on a given cell unit of the culture conditions to which it has been exposed.
10. A method according to paragraphs 3 to 8 wherein the culture conditions required to partially or fully differentiate a cell type are first identified in a method comprising the steps of:
   (a) providing a first-set of groups of cell units each comprising one or more cells, and exposing said groups to desired culture conditions;
   (b) pooling two or more of said groups to form at least one second pool;
   (c) subdividing the second pool to create a further set of groups of cell units;
   (d) exposing said further groups to desired culture conditions;
   (e) optionally, repeating steps (b) - (d) iteratively as required; and
   (f) assessing the effect on a given cell unit of the culture conditions to which it has been exposed.
11. A method according to paragraph 9 or 10 wherein cell units that are partially differentiated are then isolated and used in the method of any of claims 1 to 8.
12. Use of a partially differentiated cell type identified in paragraph 9 or 10 in the method of any preceding claim.
13. A method according to paragraphs 9 to 12 wherein cell units are labelled and the label(s) reflect(s) the culture conditions to which the cell unit has been exposed.
14. A method according to paragraph 13, wherein the label is spatially encoded.
15. A method according to paragraph 13 or 14, wherein the label is selected from the group consisting of a virus, an oligonucleotide, a peptide, a fluorescent compound, a secondary amine, a halocarbon, a mixture of stable isotopes, a bar code, an optical tag, a bead, a quantum dot and a radiofrequency encoding tag.
16. A method according to paragraph 15 wherein two or more labels are selected and used in combination to label a cell unit.
17. A method according to paragraphs 9 to 16, wherein the cells are cultured in cell units, each cell unit comprising one or more cells.
18. A method according to paragraph 9 to 16, wherein the cell units are single cells.
19. A method according to paragraph 9 to 17, wherein each cell unit comprises one or more cells adherent to or bounded by a solid substrate.
20. A method according to paragraph 19, wherein the solid substrate is a microcarrier or bead.
21. A method according to paragraph 19, wherein the solid substrate is a well or medium-permeable barrier.
22. A method according to paragraphs 9 to 21, wherein the culture conditions are media to which the cell is exposed.
23. A method according to paragraph 22, wherein the media contain one or more specific agents which influence a cellular process,
24. A method according to paragraphs 3 to 23, wherein the cell culture conditions comprise culturing at one or more specific temperatures or partial pressures of oxygen or carbon dioxide.
25. A method according to paragraphs 3 to 24, wherein the cell culture conditions comprise culturing on one or more specific substrates.
26. A method according to any preceding paragraph wherein a first cell is differentiated to a second cell type by modulating cell signalling and/or the expression of one or more genes in the cell.
27. A method according to paragraph 26, wherein modulation of gene expression in the cell comprises transfection of said one or more genes into the cell.
28. A method according to paragraph 26, wherein modulation of gene expression comprises the exogenous administration of a gene product.
29. A method according to any preceding paragraph wherein the differentiation of the cell is monitored by observing the phenotype of the cell or by detecting the modulation of expression of one or more genes in a cell, thereby determining the state of differentiation of said cell.
30. A method according to paragraph 29, wherein the modulation of expression of one or more reporter genes is observed wherein the reporter gene(s) respond(s) to one or more differentiation states of said cell.
31. A method according to paragraph 29 or 30 wherein the expression of genes involved is monitored on a gene chip.
32. A method according to paragraph 29, wherein said one or more genes encode a marker.
33. A method according to paragraph 32, wherein said marker may be detected by an immunoassay.
34. A method according to any preceding method paragraph wherein the differentiation of a cell is monitored by loss of proliferative ability.
35. A method according to paragraphs 4 or 5 wherein stem cells or cells that have been derived from stem cells in vitro, are cultured by a method comprising the steps of:
   a) combining one or more cultures of cells grown under different conditions; and
   b) culturing the cells.
36. A method according to paragraph 35, wherein said stem cells are subjected to at least one change of culture conditions.
37. A method according to paragraph 36, wherein said change of culture conditions comprises a change of medium.
38. A method according to paragraph 4 wherein differentiated cells Have been obtained from stem cells in vitro by a method comprising the steps of:
   (a) Growing stem cells adherent to microcarriers in a culture medium;
   (b) Transferring the microcarriers from one culture medium to another;
   (c) Optionally repeating step (b) as required; and
   (d) Obtaining the differentiated cells attached to the microcarrier.
39. A method according to paragraph 38, wherein stem cells or partially differentiated cells are exposed to said potential modulator whilst still attached to said microcarrier.
40. A method according to paragraph 38, wherein the differentiated cells are isolated by enzymatic detachment from the microcarrier.
41. A method according to paragraphs 38, 39 or 40 wherein the process is scaled up such that at least 50g (dry weight) of microcarrier is employed.
42. A method-according to paragraph 38 or 41, wherein the differentiated cells are isolated by digestion of the microcarrier.
43. The method according to paragraph 4 wherein pluripotent stem cells have been grown in vitro by a method comprising the steps of:
   (a) seeding said cells on microcarriers; and
   (b) propagating the cells while attached to the carriers.
44. A method according to any preceding paragraph in which the potential modulator comprises an organic or inorganic small molecule, a natural or derivatised carbohydrate, protein, polypeptide, peptide, glycoprotein, nucleic acid, DNA, RNA, oligonucleotide or protein-nucleic acid (PNA).
45. A method according to any preceding paragraph wherein the potential modulator is obtained from a library of small molecules with drug like properties.
46. Use of a compound library to identify a potential modulator according to any preceding paragraph.
47. A pharmaceutical composition comprising a modulator identified according to any preceding paragraph together with a pharmaceutically acceptable carrier; diluent or expient.
48. Use of a modulator identified in any preceding paragraph in the manufacture of a medicament for treatment of a disease.
49. Use of a modulator according to paragraph 48 wherein the treatment is a cell replacement therapy.
50. A partially differentiated cell, which has been differentiated in vitro from a stem cell and arrested along a differentiation pathway between a stem cell and a differentiated cell type.

The invention will now be further described by way of an Example, which is meant to serve to assist one of ordinary skill in the art in carrying out the invention and is not intended in any way to limit the scope of the invention.

### Example

A screen was performed to identify regenerative drugs capable of stimulating the myeloid lineages of the haematopoietic system. The screen was performed by using a series of culture steps to differentiate mouse embryonic stem cells (mESC) seeded on microcarriers into progenitors of the myeloid lineages, and subsequently subjecting these to culture conditions in the presence or absence of the lineage specific haematopoietic growth factors TPO, M-CSF and IL-5, or the control compound Vitamin C which is reported to affect cell survival but is not a haematopoietic growth factor. After 48 hours the effect on myeloid cell differentiation was assessed using the appearance of macrophages as a surrogate assay. This screen identified TPO and M-CSF as haematopoietio regenerative drugs capable of stimulating the myeloid lineage.

### Materials and methods

### Reagents

murine stem-cell factor (SCF) (R&D Systems)
murine thrombopoietin (TPO) (R&D Systems)
human erythropoietin (EPO) (R&D Systems)
human interleukin 6 (IL-6) (R&D Systems)
human transforming growth-factor β1 (TGFβ-1) (R&D Systems)
murine macrophage colony stimulating factor (M-CSF) (R&D Systems)
murine interleukin 3 (IL-3) (R&D Systems)
retinoic acid (Sigma)
human bone morphogenetic protein 2 (BMP2) (R&D Systems)
mouse interleukin 5 (IL-5) ((R&D Systems)
Ascorbic acid (Vitamin C) (Sigma)

### Microculture of mESC

CultiSpher-G microcarriers (Percell Biolytica AB) were hydrated and sterilized according to the manufacturer's recommendations.

D3 ES cells (ATTC no. CRL-1934) were grown on gelatine-coated plastic in KO-DMEM containing 15% knock-out serum replacement (KOSR), 1% non-essential amino acids (NEAA) 1% Glutamax, 0.5% penicillin/streptomycin, 0.1 mM □ mercaptoethanol (β-ME; Sigma) and 1000U/ml Leukemia Inhibitory Factor (LIF; Chemicon); all from Invitrogen unless indicated otherwise.

On the day preceding day 1 of the experiment, approximately 1.5 x 10⁴ biotinylated micocarriers equilibrated in medium A (IMDM (Gibco), 15% KOSR, 1% NEAA, 0.5% pen/strep, 0.1mM β-ME, 1000U/ml LIF and 1.5x10⁻⁴M 1-thioglycerol (MTG; Stigma)) were added to 100ml of medium A containing approximately 4.5 x 10⁶ ES cells, split into three equal aliquots placed in wells of a 100mm square petri dish (Bibby Sterilin) and incubated overnight.

### Preparation of progenitors from mESC

In order to prepare myeloid progenitors a two step method was employed. Beads seeded with mESC as described above were incubated in IMDM containing 10⁻⁸M retinoic acid for 72h. The beads were then washed in PBS and transferred to Stemline™ Haematopoietic Expansion Medium (Sigma) containing 40ng/ml SCF, 2.5ng TGF-β1-, 5ng/ml BMP2 and 20nd/ml TPO and incubated for 48h.

### Cell-based assay for regenerative drugs

Beads bearing myeloid progenitors prepared as described above were mixed, washed in PBS and transferred to the Test Growth Medium (Stemline™ Haematopoietic Expansion Medium (Sigma) supplemented with 30ng/ml IL-3 and 20ng/ml IL-6). Equal aliquots of approximately 100 beads were dispensed into the wells of a 48 well plate and incubated in the presence or absence of 50μg/ml Vitamin C, 10ng/ml IL-5, 20ng/ml TPO and 20ng/ml M-CSF. The screen was carried out in triplicate in wells of separate plates placed in the same incubator.

After 48h, 1mg of the macrophage assay reagent DQ-ovalbumin (Molecular Probes) was-made up in 0.4ml PBS and added to each well at a dilution of 1:100. Following incubation for at least 4h, the medium was aspirated and replayed with PBS. The samples were examined on a Nikon TE2000-S inverted epifluorescent microscope using a FITC filter set to quahtitate microcarriers bearing large, round cells internally labelled With green fluorescence.

The results of the screen are reported as the average number (per cent) of microcarriers that were decorated with macrophage. In Test Growth Medium alone (i.e. in the absence of any test reagents) the average conversion to macrophage was 1%, which was taken as the basal level of conversion qwing to spontaneous differentiation. When the negative control compound Vitamin C Was used as the test reagent the average conversion to macrophage was 0%, i.e. below the basal level, indicating it had no influence on differentiation. When the haematopoietic growth factor IL-5 was used as the test reagent the average conversion to macrophage was 0%, i.e. also below the basal level, indicating it had no influence on differentiation of myeloid cells in this assay. IL-5 is known to influence the lymphoid haematopoietic lineage but has no notable effects on the myeloid branch. However, when either TPO or M-CSF was used as the test reagent, the average conversion to macrophage was increased to 6%, representing a significant increase over background.

This mESC-based assay is therefore capable of identifying reagents which act to differentiate myeloid progenitors and could therefore be used to screen libraries of chemical compounds to identify novel regenerative drugs.

## Claims

1. A method for identifying a potential modulator of a cell signalling pathway in a screen to identify a regenerative drug, wherein the regenerative drug acts on a progenitor cell to form a cell for repair or regeneration of a tissue or organ in a body, comprising the steps of:
(a) providing a cell of a first cell type, wherein said first cell type is differentiated to a second cell type via a progenitor cell;
(b) deriving said progenitor cell *in vitro,* by sequentially exposing said first cell type to one or more culture media;
(c) exposing the derived progenitor cell to said potential modulator; and
(d) monitoring the differentiation to determine formation of the second cell type.

2. The method according to claim 1 for identifying a regenerative drug that acts on a progenitor cell to form a cell for repair or regeneration of a tissue or organ in a body, comprising the steps of:
(a) providing a cell of a first cell type which is differentiated to a cell of a second cell type via a progenitor cell, wherein the tissue or organ to be repaired or regenerated comprises cells of the second cell type;
(b) deriving said progenitor cell *in vitro,* by sequentially exposing said first cell type to one or more culture media;
(c) exposing the derived progenitor cell to said potential modulator; and
(d) monitoring the differentiation to determine formation of the second cell type.

3. The method according to claim 1 or 2 wherein the first cell type is obtained or obtainable from an embryo or foetus, excluding a human embryo, and optionally modified to allow amplification.

4. The method according to claim 1, 2 or 3, wherein the first cell type is a self-renewing stem cell.

5. The method according to any of the preceding claims, wherein the differentiation steps are carried out on cells which are part of a cell unit, preferably wherein the cell unit comprises a microcarrier or other scaffold.

6. The method according to any of the preceding claims, wherein the progenitor cell is a cell which has been arrested along a differentiation pathway between a stem cell and a differentiated cell type.

7. The method according to any of the preceding claims wherein the first cell type is a primary cell.

8. The method according to any of the preceding claims wherein the tissue of origin of the cell type is selected from a group consisting of brain, heart, liver, lung, hair, eye, gut, blood, ear, kidney, skin, tooth, pancreas, muscle, bone and vasculature.

9. The method according to any of the preceding claims, wherein the potential modulator is an inhibitor or a promoter of a cell signalling pathway.

10. The method according to any of the preceding claims wherein a first cell is differentiated to a second cell type by modulating cell signalling and/or the expression of one or more genes in the cell.

11. The method according to claim 10, wherein modulation of gene expression in the cell comprises transfection of said one or more genes into the cell or wherein modulation of gene expression comprises the exogenous administration of a gene product.

12. The method according to any of the preceding claims wherein the differentiation of the first cell type or the progenitor cell is monitored by observing the phenotype of the cell or by detecting the modulation of expression of one or more genes in a cell, thereby determining the state of differentiation of said cell.

13. The method according to claim 12, wherein the modulation of expression of one or more reporter genes is observed wherein the reporter gene(s) respond(s) to one or more differentiation states of said cell.

14. The method according to claim 12 or claim 13, wherein said one or more genes encode a marker.

15. The method according to claim 14, wherein said marker may be detected by an immunoassay.

16. The method according to any of the preceding claims wherein the differentiation of a cell is monitored by loss of proliferative ability.

17. The method according to any preceding claim wherein the potential modulator comprises an organic or inorganic small molecule, a natural or derivatised carbohydrate, protein, polypeptide, peptide, glycoprotein, nucleic acid, DNA, RNA, oligonucleotide or protein-nucleic acid (PNA).

18. The method according to any of the preceding claims wherein the potential modulator is obtained or obtainable from a library of small molecules with drug like properties.

19. The method according to any of the preceding claims, wherein the progenitor cell is a physiologically relevant progenitor cell obtainable by:-
(a) identifying the cell of the second type, referred to as a target phenotype of interest and being a cell of the tissue or organ;
(b) obtaining cells of a first type;
(c) determining a differentiation protocol which leads to the appearance of the target phenotype from cells of the first type via a progenitor cell; and
(d) modifying the protocol such that the differentiation process is stalled at a stage in which the progenitor cells are present, thereby providing said physiologically relevant progenitor cells.

20. The method according to any preceding claim, wherein the progenitor cell is a lineage-committed, bipotent or unipotent progenitor cell.

21. The method of claim 20, wherein the progenitor cell is a lineage-committed, unipotent progenitor cell.

22. The method according to any preceding claim, wherein the progenitor cell has a limited proliferation capacity.

23. The method according to any preceding claim, wherein the screen is used in drug discovery and comprises lead compound discovery, selection and optimization.

24. The method according to any preceding claim, wherein the regenerative drug is for *in vivo* administration.

25. The method according to any of claims 1-23, wherein the regenerative drug is for ex *vivo* administration.

26. A method for identifying a regenerative drug, wherein the regenerative drug acts on a progenitor cell to form a cell for repair or regeneration of a tissue or organ in a body, comprising the use of a progenitor cell, wherein said progenitor cell is differentiated from a first cell type *in vitro* by sequentially exposing said first cell type to two or more culture media.

27. Use of a progenitor cell in a drug screening assay to identify a regenerative drug, wherein the regenerative drug acts on a progenitor cell to form a cell for repair or regeneration of a tissue or organ in a body, wherein said progenitor cell is differentiated from a first cell type *in vitro* by sequentially exposing said first cell type to two or more culture media.

## Patentansprüche

1. Verfahren zum Identifizieren eines potentiellen Modulators eines zellensignalisierenden Pfades in einem Screening, um ein regeneratives Medikament zu identifizieren, wobei das regenerative Medikament an einer Vorläuferzelle wirkt, um eine Zelle zum Reparieren oder Regenerieren eines Gewebes oder Organs in einem Körper zu bilden, umfassend die folgenden Schritte:
(a) Bereitstellen einer Zelle eines ersten Zelltyps, wobei der erste Zelltyp über eine Vorläuferzelle von einem zweiten Typ differenziert ist;
(b) Gewinnen der Vorläuferzelle *in vitro*, indem der erste Zelltyp sequentiell einem oder mehreren Kulturmedien ausgesetzt wird;
(c) Aussetzen der gewonnenen Vorläuferzelle gegenüber dem potentiellen Modulator; und
(d) Überwachen der Differenzierung, um Bildung des zweiten Zelltyps zu bestimmen.

2. Verfahren nach Anspruch 1 zum Identifizieren eines regenerativen Medikaments, das an einer Vorläuferzelle wirkt, um eine Zelle zum Reparieren oder Regenerieren eines Gewebes oder Organs in einem Körper zu bilden, umfassend die folgenden Schritte:
(a) Bereitstellen einer Zelle eines ersten Zelltyps, die über eine Vorläuferzelle von einer Zelle eines zweiten Zelltyps differenziert ist, wobei das zu reparierende Gewebe oder Organ Zellen des zweiten Zelltyps umfasst;
(b) Gewinnen der Vorläuferzelle *in vitro*, indem der erste Zelltyp sequentiell einem oder mehreren Kulturmedien ausgesetzt wird;
(c) Aussetzen der gewonnenen Vorläuferzelle gegenüber dem potentiellen Modulator; und
(d) Überwachen der Differenzierung, um Bildung des zweiten Zelltyps zu bestimmen.

3. Verfahren nach Anspruch 1 oder 2, wobei der erste Zelltyp von einem Embryo oder Fötus gewonnen wird oder gewinnbar ist, außer von einem menschlichen Embryo, und gegebenenfalls modifiziert wird, um Amplifikation zu ermöglichen.

4. Verfahren nach Anspruch 1, 2 oder 3, wobei der erste Zelltyp eine sich selbst erneuernde Stammzelle ist.

5. Verfahren nach einem der vorhergehenden Ansprüche, wobei die Differenzierungsschritte an Zellen ausgeführt werden, die Teil einer Zelleinheit sind, wobei die Zelleinheit bevorzugt einen Mikroträger oder ein anderes Gerüst umfasst.

6. Verfahren nach einem der vorhergehenden Ansprüche, wobei die Vorläuferzelle eine Zelle ist, die entlang eines Differenzierungspfades zwischen einer Stammzelle und einem differenzierten Zelltyp aufgehalten wurde.

7. Verfahren nach einem der vorhergehenden Ansprüche, wobei der erste Zelltyp eine Primärzelle ist.

8. Verfahren nach einem der vorhergehenden Ansprüche, wobei das Ursprungsgewebe des Zelltyps ausgewählt ist aus einer Gruppe bestehend aus Gehirn, Herz, Leber, Lunge, Haar, Auge, Darm, Blut, Ohr, Niere, Hals, Zahn, Pankreas, Muskel, Knochen und Gefäß.

9. Verfahren nach einem der vorhergehenden Ansprüche, wobei der potentielle Modulator ein Inhibitor oder ein Promotor eines zellensignalisierenden Pfades sein kann.

10. Verfahren nach einem der vorhergehenden Ansprüche, wobei eine erste Zelle durch das Modulieren von Zellensignalgebung und/oder die Expression eines oder mehrerer Gene in der Zelle von einem zweiten Zelltyp differenziert wird.

11. Verfahren nach Anspruch 10, wobei die Modulation der Genexpression in der Zelle die Transfektion des einen oder der mehreren Gene in die Zelle umfasst oder wobei Modulation von Genexpression die exogene Verabreichung eines Genprodukts umfasst.

12. Verfahren nach einem der vorhergehenden Ansprüche, wobei die Differenzierung des ersten Zelltyps oder die Vorläuferzelle durch das Beobachten des Phänotyps der Zelle oder durch das Detektieren der Modulation der Expression eines oder mehrerer Gene in einer Zelle überwacht wird, wodurch das Stadium der Differenzierung in der Zelle bestimmt wird.

13. Verfahren nach Anspruch 12, wobei die Modulation der Expression eines oder mehrerer Reportergene beobachtet wird, wobei das/die Reportergen(e) auf einen oder mehrere Differenzierungsstadien der Zelle antwortet/antworten.

14. Verfahren nach Anspruch 12 oder Anspruch 13, wobei eine oder mehrere Gene einen Marker codieren.

15. Verfahren nach Anspruch 14, wobei der Marker durch einen Immunassay detektiert werden kann.

16. Verfahren nach einem der vorhergehenden Ansprüche, wobei die Differenzierung einer Zelle über den Verlust von proliferativer Fähigkeit überwacht wird.

17. Verfahren nach einem der vorhergehenden Ansprüche, wobei der potentielle Modulator ein organisches oder anorganisches Kleinmolekül, ein natürliches oder derivatisiertes Kohlenhydrat, Protein, Polypeptid, Peptide, Glycoprotein, Nukleinsäure, DNA, RNA, Oligonukleotid oder Proteinnukleinsäure (PNA) umfasst.

18. Verfahren nach einem der vorhergehenden Ansprüche, wobei der potentielle Modulator aus einer Bibliothek von Kleinmolekülen mit medikamentenähnlichen Eigenschaften gewonnen wird oder gewinnbar ist.

19. Verfahren nach einem der vorhergehenden Ansprüche, wobei die Vorläuferzelle eine physiologisch relevante Vorläuferzelle ist, die gewinnbar ist durch:
(a) Identifizieren der Zelle des zweiten Typs, der als Zielphänotyp von Interesse bezeichnet wird und eine Zelle des Gewebes oder Organs ist;
(b) Gewinnen von Zellen eines ersten Typs;
(c) Bestimmen eines Differenzierungsprotokolls, das über eine Vorläuferzelle zum Erscheinen des Zielphänotyps von Zellen des ersten Typs führt; und
(d) Modifizieren des Protokolls, so dass der Differenzierungsvorgang auf einer Stufe, in der die Vorläuferzellen vorhanden sind, verzögert wird, wodurch die physiologisch relevanten Vorläuferzellen bereitgestellt werden.

20. Verfahren nach einem der vorhergehenden Ansprüche, wobei die Vorläuferzelle eine abstammungsgebundene bipotente oder unipotente Vorläuferzelle ist.

21. Verfahren nach Anspruch 20, wobei die Vorläuferzelle eine abstammungsgebundene, unipotente Vorläuferzelle ist.

22. Verfahren nach einem der vorhergehenden Ansprüche, wobei die Vorläuferzelle eine beschränkte Proliferationsfähigkeit aufweist.

23. Verfahren nach einem der vorhergehenden Ansprüche, wobei das Screening für die Medikamentenentdeckung verwendet wird und Leitkomponentenentdeckung, -selektion und -optimierung umfasst.

24. Verfahren nach einem der vorhergehenden Ansprüche, wobei das regenerative Medikament zur Verabreichung *in vivo* vorgesehen ist.

25. Verfahren nach einem der Ansprüche 1-23, wobei das regenerative Medikament zur Verabreichung *ex vivo* vorgesehen ist.

26. Verfahren zum Identifizieren eines regenerativen Medikaments, wobei das regenerative Medikament an einer Vorläuferzelle wirkt, um eine Zelle zur Reparatur oder Regeneration eines Gewebes oder Organs in einem Körper zu bilden, umfassend die Verwendung einer Vorläuferzelle, wobei die Vorläuferzelle von dem ersten Zelltyp *in vitro* differenziert wird, indem der erste Zelltyp sequentiell den zwei oder mehr Kulturmedien ausgesetzt wird.

27. Verwenden einer Vorläuferzelle bei einem Medikamenten-Screening-Assay zum Identifizieren eines regenerativen Medikaments, wobei das regenerative Medikament an einer Vorläuferzelle wirkt, um eine Zelle zur Reparatur oder Regeneration eines Gewebes oder Organs in einem Körper zu bilden, wobei die Vorläuferzelle von dem ersten Zelltyp *in vitro* differenziert wird, indem der erste Zelltyp sequentiell den zwei oder mehr Kulturmedien ausgesetzt wird.

## Revendications

1. Méthode d'identification d'un modulateur potentiel d'une voie de signalisation cellulaire lors d'un criblage permettant d'identifier un médicament régénérateur, dans laquelle le médicament régénérateur agit sur une cellule progénitrice afin de former une cellule de réparation ou de régénération d'un tissu ou d'un organe d'un corps, comprenant des étapes consistant à :
(a) utiliser une cellule d'un premier type cellulaire, dans laquelle ledit premier type cellulaire est différencié d'un second type cellulaire par le biais d'une cellule progénitrice ;
(b) dériver ladite cellule progénitrice *in vitro*, par exposition séquentielle dudit premier type cellulaire à un ou plusieurs milieux de culture ;
(c) exposer la cellule progénitrice dérivée audit modulateur potentiel ; et
(d) surveiller la différenciation pour déterminer la formation du second type cellulaire.

2. Méthode selon la revendication 1 d'identification d'un médicament régénérateur qui agit sur une cellule progénitrice afin de former une cellule de réparation ou de régénération d'un tissu ou d'un organe d'un corps, comprenant des étapes consistant à :
(a) utiliser une cellule d'un premier type cellulaire qui est différenciée d'une cellule d'un second type cellulaire par le biais d'une cellule progénitrice, dans laquelle le tissu ou l'organe à réparer ou à régénérer comprend des cellules du second type cellulaire ;
(b) dériver ladite cellule progénitrice *in vitro*, par exposition séquentielle dudit premier type cellulaire à un ou plusieurs milieux de culture ;
(c) exposer la cellule progénitrice dérivée audit modulateur potentiel ; et
(d) surveiller la différenciation pour déterminer la formation du second type cellulaire.

3. Méthode selon la revendication 1 ou 2, dans laquelle le premier type cellulaire est obtenu, on peut être obtenu, à partir d'un embryon ou d'un foetus, à l'exception d'un embryon humain, et éventuellement modifié pour permettre une amplification.

4. Méthode selon la revendication 1, 2 ou 3, dans laquelle le premier type cellulaire est une cellule souche auto-renouvelée.

5. Méthode selon l'une quelconque des revendications précédentes, dans laquelle les étapes de différenciation sont exécutées sur des cellules qui font partie d'une unité cellulaire, de préférence dans laquelle l'unité cellulaire comprend un microsupport ou un échafaudage autre.

6. Méthode selon l'une quelconque des revendications précédentes, dans laquelle la cellule progénitrice est une cellule qui a été arrêtée le long d'une voie de différenciation entre une cellule souche et un type cellulaire différencié.

7. Méthode selon l'une quelconque des revendications précédentes, dans laquelle le premier type cellulaire est une cellule primaire.

8. Méthode selon l'une quelconque des revendications précédentes, dans laquelle le tissu d'origine du type cellulaire est sélectionné à partir d'organes d'un groupe d'organes constitué d'un cerveau, d'un coeur, d'un foie, d'un poumon, d'un cheveu, d'un oeil, d'un intestin, de sang, d'une oreille, d'un rein, de peau, d'une dent, d'un pancréas, d'un muscle, d'un os et d'une vasculature.

9. Méthode selon l'une quelconque des revendications précédentes, dans laquelle le modulateur potentiel est un inhibiteur ou un promoteur d'une voie de signalisation cellulaire.

10. Méthode selon l'une quelconque des revendications précédentes, dans laquelle une première cellule est différenciée d'un second type cellulaire par modulation de signalisation cellulaire et/ou de l'expression d'un ou de plusieurs gènes de la cellule.

11. Méthode selon la revendication 10, dans laquelle la modulation d'expression génique de la cellule comprend une transfection dudit un ou desdits plusieurs gènes dans la cellule, ou dans laquelle la modulation d'expression génique comprend l'administration exogène d'un produit génique.

12. Méthode selon l'une quelconque des revendications précédentes, dans laquelle la différenciation du premier type cellulaire ou de la cellule progénitrice est surveillé par observation du phénotype de la cellule ou par détection de la modulation d'expression d'un ou de plusieurs gènes d'une cellule, déterminant ainsi l'état de différenciation de ladite cellule.

13. Méthode selon la revendication 12, dans laquelle la modulation d'expression d'un ou de plusieurs gènes rapporteurs est observée, dans laquelle le ou les gènes rapporteurs répondent à un ou plusieurs états de différenciation de ladite cellule.

14. Méthode selon la revendication 12 ou la revendication 13, dans laquelle ledit un ou lesdits plusieurs gènes codent un marqueur.

15. Méthode selon la revendication 14, dans laquelle ledit marqueur peut être détecté par un immuno-essai.

16. Méthode selon l'une quelconque des revendications précédentes, dans laquelle la différenciation d'une cellule est surveillée par perte de capacité de prolifération.

17. Méthode selon l'une quelconque des revendications précédentes, dans laquelle le modulateur potentiel comprend une petite molécule organique ou inorganique, un hydrate de carbone naturel ou dérivé, une protéine, un polypeptide, un peptide, une glycoprotéine, un acide nucléique, un ADN, un ARN, un oligonucléotide ou un acide nucléique protéique (ANP).

18. Méthode selon l'une quelconque des revendications précédentes, dans laquelle le modulateur potentiel est obtenu, ou peut être obtenu, à partir d'une bibliothèque de petites molécules ayant des propriétés similaires à celles d'un médicament.

19. Méthode selon l'une quelconque des revendications précédentes, dans laquelle la cellule progénitrice est une cellule progénitrice d'intérêt physiologique pouvant être obtenue par :
(a) identification de la cellule du second type, que l'on appelle phénotype cible d'intérêt et qui est une cellule du tissu ou de l'organe ;
(b) obtention de cellules d'un premier type ;
(c) détermination d'un protocole de différenciation qui mène à l'apparition du phénotype cible à partir de cellules du premier type par le biais d'une cellule progénitrice ; et
(d) modification du protocole de sorte que le processus de différenciation soit bloqué à un étage dans lequel sont présentes les cellules progénitrices, fournissant ainsi lesdites cellules progénitrices d'intérêt physiologique.

20. Méthode selon l'une quelconque des revendications précédentes, dans laquelle la cellule progénitrice est une cellule progénitrice à lignée déterminée, bipotente ou unipotente.

21. Méthode selon la revendication 20, dans laquelle la cellule progénitrice est une cellule progénitrice à lignée déterminée, unipotente.

22. Méthode selon l'une quelconque des revendications précédentes, dans laquelle la cellule progénitrice a une capacité de prolifération limitée.

23. Méthode selon l'une quelconque des revendications précédentes, dans laquelle le criblage est utilisé pour la découverte de médicaments et comprend la découverte, la sélection et l'optimisation de composés têtes de série.

24. Méthode selon l'une quelconque des revendications précédentes, dans laquelle le médicament régénérateur est prévu pour une administration *in vivo*.

25. Méthode selon l'une quelconque des revendications 1 à 23, dans laquelle le médicament régénérateur est prévu pour une administration *ex vivo*.

26. Méthode permettant d'identifier un médicament régénérateur, dans laquelle le médicament régénérateur agit sur une cellule progénitrice pour former une cellule de réparation ou de régénération d'un tissu ou d'un organe d'un corps, consistant à utiliser une cellule progénitrice, dans laquelle ladite cellule progénitrice est différenciée d'un premier type cellulaire *in vitro* par exposition séquentielle dudit premier type cellulaire à deux milieux de culture, ou plus.

27. Utilisation d'une cellule progénitrice lors d'un essai de criblage de médicament permettant d'identifier un médicament régénérateur, dans laquelle le médicament régénérateur agit sur une cellule progénitrice pour former une cellule de réparation ou de régénération d'un tissu ou d'un organe d'un corps, dans laquelle ladite cellule progénitrice est différenciée d'un premier type cellulaire *in vitro* par exposition séquentielle dudit premier type cellulaire à deux milieux de culture, ou plus.
